# EUROPEAN PATENT APPLICATION

(11) **EP 4 241 664 A1**
(43) Date of publication of application: **13.09.2023**
(21) Application number: 21909142.8
(22) Date of filing: 08.12.2021
(51) Int. Cl.: A61B 5/0205, A61B 5/00

(54) **PHYSIOLOGICAL PARAMETER MEASUREMENT METHOD, AND ELECTRONIC DEVICE**

(30) Priority: 22.12.2020 CN 202011529749
(71) Applicant: Huawei Technologies Co., Ltd., Shenzhen, Guangdong 518129 (CN)
(72) Inventor: PENG, Jiahui, Shenzhen, Guangdong 518129 (CN); HUANG, Xi, Shenzhen, Guangdong 518129 (CN); QIU, Lingzhi, Shenzhen, Guangdong 518129 (CN); WU, Lian, Shenzhen, Guangdong 518129 (CN)
(74) Representative: MERH-IP Matias Erny Reichl Hoffmann Patentanwälte PartG mbB
(86) International application number: PCT/CN2021/136277
(87) International publication number: WO 2022/135145

(57) **Abstract**

A method for measuring a physiological parameter and an electronic device are provided. The electronic device may be a wearable device integrated with an ACC sensor, a PPG sensor, and/or an ECG sensor. In the method, a plurality of physiological parameters of a user may be measured at a time by using physiological parameter measurement software. For example, at least two physiological parameters of atrial fibrillation, premature beat, a heart rate, arteriosclerosis, and blood oxygen may be measured at a time. The method has high measurement efficiency, a simple user operation, and good user experience.

## Description

This application claims priority to Chinese Patent Application No. 202011529749.7, filed with the China National Intellectual Property Administration on December 22, 2020 and entitled "METHOD FOR MEASURING PHYSIOLOGICAL PARAMETER AND ELECTRONIC DEVICE", which is incorporated herein by reference in its entirety.

### TECHNICAL FIELD

This application relates to the field of electronic devices, and more specifically, to a method for measuring a physiological parameter and an electronic device.

### BACKGROUND

With development of electronic devices, a user has an increasingly high requirement on functions of the electronic devices. For example, a wearable device may not only serve as a clock, but also measure a physiological parameter of the user. For example, a wearable device may measure physiological parameters such as atrial fibrillation, premature beat, and arteriosclerosis.

For an existing electronic device, when a user wants to measure a plurality of physiological parameters, the user needs to measure each physiological parameter once. An operation is relatively complex, and user experience is poor. In addition, measurement of physiological parameters is independent of each other, and data is not shared. Consequently, measurement time is relatively long, and user experience is poor.

### SUMMARY

This application provides a method for measuring a physiological parameter and an electronic device, so that a plurality of physiological parameters of a user can be measured at a time, a user operation is simple, measurement efficiency is high, and user experience is relatively good.

According to a first aspect, a method for measuring a physiological parameter is provided. The method is applied to an electronic device including a physiological parameter sensor, and the method includes: displaying a physiological parameter measurement interface, where the physiological parameter measurement interface includes a plurality of physiological parameter identifiers; receiving a physiological parameter measurement operation, where the physiological parameter measurement operation includes an operation of selecting a first physiological parameter identifier and a second physiological parameter identifier, and the first physiological parameter identifier and the second physiological parameter identifier are physiological parameter identifiers in the plurality of physiological parameter identifiers; and measuring a first physiological parameter and a second physiological parameter of a measured object by using the physiological parameter sensor based on the physiological parameter measurement operation.

It should be understood that a corresponding physiological parameter may be measured by using the physiological parameter sensor.

A type of the physiological parameter sensor and a type of the physiological parameter are not limited in this embodiment of this application.

For example, the physiological parameter sensor may include at least one of the following: an accelerometer (accelerometer, ACC) sensor, a photoplehysmography (photo plethysmo graphy, PPG) sensor, and an electrocardiography (Electrocardiography, ECG) sensor.

For example, the physiological parameter may be a physiological parameter related to an arrhythmia characteristic, a physiological parameter related to a vascular characteristic, a physiological parameter related to a blood oxygen characteristic, a physiological parameter related to a stress characteristic, sleep, or the like.

For example, the physiological parameter related to the arrhythmia characteristic may be atrial fibrillation, premature beat, a heart rate, or the like.

For example, the physiological parameter related to the vascular characteristic may be arteriosclerosis.

In this embodiment of this application, display manners of the first physiological parameter identifier and the second physiological parameter identifier on the physiological parameter measurement interface are not limited.

In a possible implementation, the first physiological parameter identifier and the second physiological parameter identifier may be displayed on the physiological parameter measurement interface in a combination manner.

For example, the first physiological parameter and the second physiological parameter are displayed in a menu option on the physiological parameter measurement interface.

In another possible implementation, the first physiological parameter identifier and the second physiological parameter identifier may be displayed on the physiological parameter measurement interface in a separate manner.

For example, the first physiological parameter identifier and the second physiological parameter identifier are displayed in two menu item options on the physiological parameter measurement interface.

Optionally, the physiological parameter measurement operation further includes an operation of selecting at least one physiological parameter identifier other than the first physiological parameter identifier and the second physiological parameter identifier. In this embodiment of this application, a user selects, by using the identifiers of the plurality of physiological parameters displayed on the physiological parameter measurement interface, the identifiers of the first physiological parameter and the second physiological parameter that need to be measured, to measure the first physiological parameter and the second physiological parameter of the measured object, so that the plurality of physiological parameters of the user can be measured at a time. Therefore, measurement efficiency is high, a user operation is simple, and user experience is relatively good.

With reference to the first aspect, in some implementations of the first aspect, the measuring a first physiological parameter and a second physiological parameter of a measured object by using the physiological parameter sensor in response to the physiological parameter measurement operation includes: collecting data of the measured object by using the physiological parameter sensor based on the physiological parameter measurement operation; extracting data related to the first physiological parameter and data related to the second physiological parameter from the collected data; obtaining the first physiological parameter based on the data related to the first physiological parameter; and obtaining the second physiological parameter based on the data related to the second physiological parameter. In a process of measuring the first physiological parameter and the second physiological parameter of the measured object, the data of the measured object is first collected by using the physiological parameter sensor, and then the data related to the first physiological parameter and the data related to the second physiological parameter are extracted from the collected data, so that corresponding physiological parameters can be obtained based on the data related to the corresponding physiological parameters, and a plurality of physiological parameters can be simultaneously measured. Therefore, measurement efficiency is high, a user operation is simple, and user experience is relatively good.

In a possible implementation, the extracted data related to the first physiological parameter is partially or completely the same as the extracted data related to the second physiological parameter.

For example, that the extracted data related to the first physiological parameter is completely the same as the extracted data related to the second physiological parameter may be understood as that a type of the data related to the first physiological parameter is the same as a type of the data related to the second physiological parameter, and a data volume of the data related to the first physiological parameter is the same as a data volume of the data related to the second physiological parameter.

For example, that the extracted data related to the first physiological parameter is partially the same as the extracted data related to the second physiological parameter may be understood as that a type of the data related to the first physiological parameter is the same as a type of the data related to the second physiological parameter, and a data volume of the data related to the first physiological parameter is different from a data volume of the data related to the second physiological parameter.

A plurality of pieces of data related to the physiological parameters may be obtained at a time from the data of the measured object collected by using the physiological parameter sensor. Therefore, data is reused, measurement efficiency is high, and user experience is good.

With reference to the first aspect, in some implementations of the first aspect, after the collecting data of the measured object by using the physiological parameter sensor based on the physiological parameter measurement operation, the method further includes: determining whether the data meets a data quality requirement; and when the data does not meet the data quality requirement, re-collecting data of the measured object by using the physiological parameter sensor, and updating the data with the re-collected data until the data meets the data quality requirement.

For example, whether the data meets the data quality requirement may be determined by using the collected data of the measured object and with reference to at least one of a wearing status of the electronic device, an ECG lead status, or a motion status of the user.

Whether the data collected by using the physiological parameter sensor meets the data quality requirement is determined, and when the collected data does not meet the data quality requirement, the data of the measured object is re-collected by using the physiological parameter sensor until the data meets the data quality requirement, thereby improving measurement accuracy of the physiological parameter.

With reference to the first aspect, in some implementations of the first aspect, when the data does not meet the data quality requirement, the method further includes: displaying prompt information, where the prompt information is used to prompt the user to correctly wear the electronic device and/or is used to prompt the user to correctly operate the electronic device.

With reference to the first aspect, in some implementations of the first aspect, the obtaining the first physiological parameter based on the data related to the first physiological parameter includes: extracting, based on the data related to the first physiological parameter, feature data related to the first physiological parameter; determining whether the feature data of the first physiological parameter meets a requirement for analyzing the first physiological parameter; and obtaining the first physiological parameter based on the feature data related to the first physiological parameter when the feature data of the first physiological parameter meets the requirement for analyzing the first physiological parameter.

With reference to the first aspect, in some implementations of the first aspect, the determining whether the feature data of the first physiological parameter meets a requirement for analyzing the first physiological parameter includes: determining whether the feature data of the first physiological parameter meets the requirement for analyzing the first physiological parameter; and determining whether a data volume corresponding to the feature data of the first physiological parameter meets the requirement for analyzing the first physiological parameter when the feature data of the first physiological parameter meets the requirement for analyzing the first physiological parameter; and the obtaining the first physiological parameter based on the feature data related to the first physiological parameter when the feature data of the first physiological parameter meets the requirement for analyzing the first physiological parameter includes: obtaining the first physiological parameter based on the feature data related to the first physiological parameter when the data volume corresponding to the feature data of the first physiological parameter meets the requirement for analyzing the first physiological parameter.

With reference to the first aspect, in some implementations of the first aspect, after the feature data of the first physiological parameter meets the requirement for analyzing the first physiological parameter, the method further includes: performing data processing on the feature data of the first physiological parameter; and the determining whether a data volume corresponding to the feature data of the first physiological parameter meets the requirement for analyzing the first physiological parameter when the feature data of the first physiological parameter meets the requirement for analyzing the first physiological parameter includes: determining whether the data volume corresponding to the feature data of the first physiological parameter meets the requirement for analyzing the first physiological parameter when processed feature data of the first physiological parameter meets the requirement for analyzing the first physiological parameter.

With reference to the first aspect, in some implementations of the first aspect, the obtaining the second physiological parameter based on the data related to the second physiological parameter includes: extracting, based on the data related to the second physiological parameter, feature data related to the second physiological parameter; determining whether the feature data of the second physiological parameter meets a requirement for analyzing the second physiological parameter; and obtaining the second physiological parameter based on the feature data related to the second physiological parameter when the feature data of the second physiological parameter meets the requirement for analyzing the second physiological parameter.

With reference to the first aspect, in some implementations of the first aspect, the determining whether the feature data of the second physiological parameter meets a requirement for analyzing the second physiological parameter includes: determining whether the feature data of the second physiological parameter meets the requirement for analyzing the second physiological parameter; and determining whether a data volume corresponding to the feature data of the second physiological parameter meets the requirement for analyzing the second physiological parameter when the feature data of the second physiological parameter meets the requirement for analyzing the second physiological parameter; and the obtaining the second physiological parameter based on the feature data related to the second physiological parameter when the feature data of the second physiological parameter meets the requirement for analyzing the second physiological parameter includes: obtaining the second physiological parameter based on the feature data related to the second physiological parameter when the data volume corresponding to the feature data of the second physiological parameter meets the requirement for analyzing the second physiological parameter.

With reference to the first aspect, in some implementations of the first aspect, after the feature data of the second physiological parameter meets the requirement for analyzing the second physiological parameter, the method further includes: performing data processing on the feature data of the second physiological parameter; and the determining whether a data volume corresponding to the feature data of the second physiological parameter meets the requirement for analyzing the second physiological parameter when the feature data of the second physiological parameter meets the requirement for analyzing the second physiological parameter includes: determining whether the data volume corresponding to the feature data of the second physiological parameter meets the requirement for analyzing the second physiological parameter when processed feature data of the second physiological parameter meets the requirement for analyzing the second physiological parameter.

With reference to the first aspect, in some implementations of the first aspect, after the measuring a first physiological parameter and a second physiological parameter of a measured object by using the physiological parameter sensor based on the physiological parameter measurement operation, the method further includes: displaying the first physiological parameter and the second physiological parameter of the measured object.

With reference to the first aspect, in some implementations of the first aspect, the displaying the first physiological parameter and the second physiological parameter of the measured object includes: displaying the first physiological parameter after measurement of the first physiological parameter is completed; and displaying the second physiological parameter after measurement of the second physiological parameter is completed.

With reference to the first aspect, in some implementations of the first aspect, the displaying the first physiological parameter and the second physiological parameter of the measured object includes: displaying the first physiological parameter and the second physiological parameter after measurement of the first physiological parameter and the second physiological parameter is completed.

According to a second aspect, a method for measuring a physiological parameter is provided. The method is applied to an electronic device including a physiological parameter sensor, and the method includes: collecting, by using the physiological parameter sensor, data that is related to a first physiological parameter and that is of a measured object; obtaining the first physiological parameter based on the data related to the first physiological parameter; detecting an operation of measuring a second physiological parameter, where the second physiological parameter is different from the first physiological parameter; and sending a measurement message to a second application in response to the operation, where the measurement message includes the data related to the first physiological parameter.

When a user measures the first physiological parameter of the measured object by using a first application, if the collected data related to the first physiological parameter may also be used as data for obtaining the second physiological parameter, when it is detected that the second physiological parameter of the measured object is measured, the data related to the first physiological parameter is sent to the second application, so that the second application can obtain the second physiological parameter without collecting the data related to the second physiological parameter, thereby being compatible with an existing physiological parameter measurement application. Therefore, data is reused, measurement efficiency is high, and user experience is relatively good.

With reference to the second aspect, in some implementations of the second aspect, after the obtaining the first physiological parameter based on the data related to the first physiological parameter, the method further includes: determining the second physiological parameter based on the data related to the first physiological parameter.

The data related to the first physiological parameter includes the data related to the second physiological parameter.

That the data related to the first physiological parameter includes the data related to the second physiological parameter may be understood as that a type of the data related to the first physiological parameter includes a type of the data related to the second physiological parameter, and a data volume of the data related to the first physiological parameter includes a data volume of the data related to the second physiological parameter.

For example, the type of the data indicates that the data is PPG data, the data is ECG data, and/or the data is ACC data.

For example, when a physiological parameter is a physiological parameter related to an arrhythmia characteristic, data related to the physiological parameter includes ACC data, PPG data, and ECG data. In addition, the data related to physiological parameter should be collected for 30 seconds.

For example, when a physiological parameter is a physiological parameter related to a vascular characteristic, data related to the physiological parameter includes ACC data, PPG data, and ECG data. In addition, the data related to physiological parameter should be collected for 60 seconds.

For example, when a physiological parameter is a physiological parameter related to a blood oxygen characteristic, data related to the physiological parameter includes ACC data and PPG data. In addition, the data related to physiological parameter should be collected for 20 seconds.

For example, when a physiological parameter is a physiological parameter related to a stress characteristic, data related to the physiological parameter includes ACC data and PPG data. In addition, the data related to physiological parameter should be collected for 60 seconds.

For example, the first physiological parameter may be a physiological parameter related to the arrhythmia characteristic, and the second physiological parameter may be a physiological parameter related to the blood oxygen characteristic. For another example, the first physiological parameter may be a physiological parameter related to the vascular characteristic, and the second physiological parameter may be a physiological parameter related to the arrhythmia characteristic, a physiological parameter related to the blood oxygen characteristic, or a physiological parameter related to the stress characteristic. For another example, the first physiological parameter may be a physiological parameter related to the stress characteristic, and the second physiological parameter may be a physiological parameter related to the blood oxygen characteristic.

With reference to the second aspect, in some implementations of the second aspect, before the detecting an operation of measuring a second physiological parameter, the method further includes: displaying or playing reminder information, where the reminder information is used to remind whether to measure the second physiological parameter.

According to a third aspect, a method for measuring a physiological parameter is provided. The method includes: receiving a measurement message from a first application, where the measurement message includes data related to a first physiological parameter; and obtaining a second physiological parameter based on the data related to the first physiological parameter, where the second physiological parameter is different from the first physiological parameter.

The second application does not need to collect data related to the second physiological parameter, and can obtain the second physiological parameter only based on the data that is related to the first physiological parameter and that is obtained from the first application. Therefore, data is reused, measurement efficiency is high, and user experience is relatively good.

According to a fourth aspect, a system is provided. The system includes a first application and a second application, where the first application is used to collect data that is related to a first physiological parameter and that is of a measured object; the first application is further configured to obtain the first physiological parameter based on the data related to the first physiological parameter; the first application is further configured to detect an operation of measuring a second physiological parameter, where the second physiological parameter is different from the first physiological parameter; the first application is further configured to send a measurement message to the second application in response to the operation, where the measurement message includes the data related to the first physiological parameter; and the second application is configured to obtain the second physiological parameter based on the data related to the first physiological parameter.

When a user measures the first physiological parameter of the measured object by using the first application, if the collected data related to the first physiological parameter may also be used as data for obtaining the second physiological parameter, when it is detected that the second physiological parameter of the measured object is measured, the data related to the first physiological parameter is sent to the second application, so that the second application can obtain the second physiological parameter without collecting the data related to the second physiological parameter. Therefore, data is reused, measurement efficiency is high, and user experience is relatively good.

With reference to the fourth aspect, in some implementations of the fourth aspect, after the first application is further configured to obtain the first physiological parameter based on the data related to the first physiological parameter, the first application is further configured to determine the second physiological parameter based on the data related to the first physiological parameter.

With reference to the fourth aspect, in some implementations of the fourth aspect, before the first application is further configured to detect an operation of measuring a second physiological parameter, the first application is further configured to display or play reminder information, where the reminder information is used to remind whether to measure the second physiological parameter.

According to a fifth aspect, an apparatus is provided. The apparatus is included in an electronic device, and the apparatus has a function of implementing any one of the first aspect to the third aspect or some implementations of the first aspect to the third aspect. The function may be implemented by hardware, or may be implemented by executing corresponding software by hardware. The hardware or the software includes one or more modules or units corresponding to the foregoing function.

According to a sixth aspect, an electronic device is provided. The electronic device includes one or more processors, a memory, and one or more computer programs. The one or more computer programs are stored in the memory. The one or more computer programs include instructions. When the instructions are executed by the electronic device, the electronic device is enabled to perform the method for measuring a physiological parameter in any one of the first aspect to the third aspect or some implementations of the first aspect to the third aspect.

According to a seventh aspect, a computer program product including instructions is provided. When the computer program product is run on an electronic device, the electronic device is enabled to perform the method for measuring a physiological parameter in any one of the first aspect to the third aspect or some implementations of the first aspect to the third aspect.

According to an eighth aspect, a computer readable storage medium is provided. The computer readable storage medium includes instructions. When the instructions are run on an electronic device, the electronic device is enabled to perform the method for measuring a physiological parameter in any one of the first aspect to the third aspect or some implementations of the first aspect to the third aspect.

It should be noted that all or some of computer program code may be stored in a first storage medium. The first storage medium may be encapsulated together with a processor, or may be encapsulated separately from a processor. This is not specifically limited in this embodiment of this application.

According to a ninth aspect, a chip is provided. The chip includes a processor and a data interface. The processor reads, by using the data interface, instructions stored in a memory, to perform the method for measuring a physiological parameter in any one of the first aspect to the third aspect or some implementations of the first aspect to the third aspect.

Optionally, in an implementation, the chip may further include a memory. The memory stores instructions. The processor is configured to execute the instructions stored in the memory. When the instructions are executed, the processor is configured to perform the method for measuring a physiological parameter in any one of the first aspect to the third aspect or some implementations of the first aspect to the third aspect.

### BRIEF DESCRIPTION OF DRAWINGS

FIG. 1 is a schematic diagram of a hardware structure of a wearable device according to an embodiment of this application;
FIG. 2 is a schematic diagram of a structure of a wearable device according to an embodiment of this application;
FIG. 3 is a schematic diagram of a graphical user interface of a wearable device according to an embodiment of this application;
FIG. 4 is a schematic diagram of a graphical user interface of a wearable device according to another embodiment of this application;
FIG. 5 is a schematic diagram of a graphical user interface of a wearable device according to still another embodiment of this application;
FIG. 6 is a schematic flowchart of a measurement method according to an embodiment of this application;
FIG. 7 is a schematic flowchart of a measurement method according to an embodiment of this application;
FIG. 8 is a schematic flowchart of a measurement method according to an embodiment of this application;
FIG. 9 is a schematic flowchart of a measurement method according to another embodiment of this application;
FIG. 10 is an example diagram of a structure of a wearable device according to an embodiment of this application;
FIG. 11 is an example diagram of a structure of a wearable device according to another embodiment of this application;
FIG. 12 is an example diagram of a structure of a wearable device according to still another embodiment of this application;
FIG. 13 is an example diagram of a structure of a wearable device according to yet another embodiment of this application; and
FIG. 14 is an example diagram of a structure of a system according to an embodiment of this application.

### DESCRIPTION OF EMBODIMENTS

Technical solutions in embodiments of this application are described in detail below with reference to the accompanying drawings of the following embodiments of this application.

In the embodiments of this application, "at least one" means one or more, and "a plurality of" means two or more. In addition, it should be understood that in descriptions of this application, terms such as "first" and "second" are merely used for distinguishing and description, but should not be understood as indicating or implying relative importance, or should not be understood as indicating or implying a sequence.

Terms used in the following embodiments are merely intended to describe specific embodiments, but are not intended to limit this application. As used in this specification and the appended claims of this application, singular expression forms "one", "one type" "said", "the foregoing", "the", and "this" are intended to also include an expression form "one or more", unless otherwise specified in the context clearly. It should be further understood that, in the embodiments of this application, "one or more" refers to one, two, or more, and "and/or" describes an association relationship between associated objects, and indicates that three relationships may exist. For example, A and/or B may indicate the following cases: Only A exists, both A and B exist, and only B exists, where A and B may be singular or plural. The character "/" generally indicates an "or" relationship between associated objects.

Reference to "an embodiment", "some embodiments", or the like described in the embodiments of this application means that one or more embodiments of this application include a specific feature, structure, or characteristic described with reference to the embodiments. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "comprise", "have", and their variants all mean "include but are not limited to", unless otherwise specifically emphasized in another manner.

A method provided in the embodiments of this application may be applied to an electronic device. The electronic device may be a wearable electronic device (also referred to as a wearable device), such as a watch, a band, a headset, or a helmet (for example, a virtual reality helmet), or may be a non-wearable device, for example, a portable electronic device with an ECG and/or PPG detection function, such as a mobile phone, a tablet computer, or a notebook computer. An example embodiment of a portable electronic device includes but is not limited to a portable electronic device using iOS^{®}, Android^{®}, Microsoft^{®}, or another operating system. It should be understood that the electronic device may not be a portable electronic device, but a desktop computer that can detect ECG and/or PPG, or the like. This is not limited in the embodiments of this application. In the following embodiments of this application, an example in which the electronic device is a wearable device is used for description.

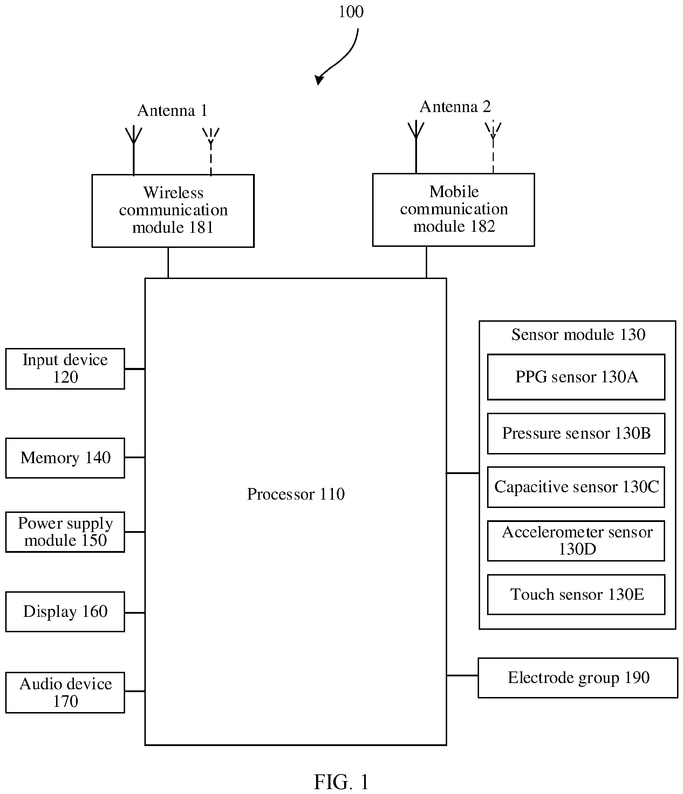

FIG. 1 is a schematic diagram of a functional block of a wearable device according to an embodiment of this application. For example, the wearable device 100 may be a smartwatch, a smart band, or the like. Referring to FIG. 1, for example, the wearable device 100 may include a processor 110, an input device 120, a sensor module 130, a memory 140, and a power supply module 150. It may be understood that components shown in FIG. 1 do not constitute a specific limitation on the wearable device 100. The wearable device 100 may further include more or fewer components than those shown in the figure, combine some components, split some components, or have different component arrangements.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, and/or a neural-network processing unit (neural-network processing unit, NPU). Different processing units may be independent devices, or may be integrated into one or more processors. The controller may be a nerve center and a command center of the wearable device 100. The controller may generate an operation control signal based on an instruction operation code and a time sequence signal, to complete control of instruction reading and instruction execution. In some other embodiments, a memory may be further disposed in the processor 110, and is configured to store instructions and data. In some embodiments, the memory in the processor 110 is a cache memory. The memory may store instructions or data that has been recently used or cyclically used by the processor 110. If the processor 110 needs to use the instructions or the data again, the processor 110 may directly invoke the instructions or the data from the memory, thereby avoiding repeated access and reducing waiting time of the processor 110, thereby improving efficiency of the wearable device 100.

The input device 120 is configured to provide user input, and may be a mechanical device. A user touches the input device 120, so that the input device 120 rotates, translates, or tilts to implement user input, to implement functions or operations such as starting (for example, powering on or off), determining or adjusting a signal (for example, adjusting a volume) of the wearable device 100.

It may be understood that the user input in this embodiment of this application may be an operation such as rotation, translation, and tilt performed by the user on the input device 120.

It may be further understood that the wearable device 100 may include one or more input devices 120.

The sensor module 130 may include one or more sensors, for example, may include a PPG sensor 130A, a pressure sensor 130B, a capacitive sensor 130C, an accelerometer sensor 130D, and a touch sensor 130E. It should be understood that FIG. 1 merely lists several examples of sensors. In actual application, the wearable device 100 may further include more or fewer sensors, or replace the listed sensors with other sensors with a same or similar function. This is not limited in this embodiment of this application.

The PPG sensor 130A may be configured to detect a heart rate, that is, a quantity of heartbeat times per unit time. In some embodiments, the PPG sensor 130A may include an optical sending unit and an optical receiving unit. The optical sending unit may irradiate a light beam into a human body (for example, a blood vessel), the light beam is reflected/refracted in a human body, and reflected/refracted light is received by the optical receiving unit, to obtain an optical signal. Because transmittance of blood changes in a fluctuation process, the emitted/refracted light changes, and an optical signal detected by the PPG sensor 130A also changes. The PPG sensor 130A may convert the optical signal into an electrical signal, to determine a heart rate corresponding to the electrical signal.

The pressure sensor 130B may be configured to detect a pressure value between a human body and the wearable device 100. The pressure sensor 130B is configured to sense a pressure signal, and may convert the pressure signal into an electrical signal. There are many types of pressure sensors 130B, such as a resistive pressure sensor, an inductive pressure sensor, and a capacitive pressure sensor. This is not limited in this embodiment of this application.

The capacitive sensor 130C may be configured to detect capacitance between two electrodes, to implement a specific function.

In some embodiments, the capacitive sensor 130C may be configured to detect capacitance between a human body and the wearable device 100. The capacitance may reflect whether the human body is in good contact with the wearable device, and may be applied to electrocardiography (Electrocardiography, ECG) detection. The human body may be used as an electrode. When the capacitive sensor 130C is disposed on an electrode of the wearable device, the capacitive sensor 130C may detect capacitance between the human body and the electrode. When the capacitance detected by the capacitive sensor 130C is excessively large or excessively small, it indicates that the human body is in relatively poor contact with the electrode. When the capacitance detected by the capacitive sensor 130C is moderate, it indicates that the human body is in relatively good contact with the electrode. Whether the human body is in good contact with the electrode affects detection of an electrical signal by the electrode, and further affects generation of the ECG. Therefore, when the wearable device 100 generates the ECG, refer to the capacitance detected by the capacitive sensor 130C.

The accelerometer sensor 130D may be configured to detect magnitudes of accelerations of the wearable device 100 in all directions (usually on three axes). The wearable device 100 is a wearable device. When the user wears the wearable device 100, the wearable device 100 moves under driving of the user. Therefore, the magnitudes of the accelerations detected by the accelerometer sensor 130D in all the directions may reflect a motion status of a human body.

The touch sensor 130E may be disposed on a display, and the touch sensor 130E and the display form a touch screen, also referred to as a "touchscreen". The touch sensor 130E is configured to detect a touch operation performed on or near the touch sensor 130E. The touch sensor 130E may transfer the detected touch operation to the processor 110, to determine a type of a touch event. The display may provide visual output related to the touch operation. In some other embodiments, the touch sensor 130E may alternatively be disposed on a surface of the display, and is at a position different from that of the display.

The memory 140 may be configured to store computer executable program code. The executable program code includes instructions. The processor 110 runs the instructions stored in the memory, to implement various function applications of the wearable device 100 and data processing. The memory 140 may include a highspeed random access memory, and may further include a non-volatile memory, such as at least one magnetic disk storage device, a flash memory device, or a universal flash storage (universal flash storage, UFS). This is not limited in this embodiment of this application.

The power supply module 150 may supply power to components such as the processor 110 and the sensor module 130 in the wearable device 100. In some embodiments, the power supply module 150 may be a battery or another portable electrical element. In some other embodiments, the wearable device 100 may be further connected to a charging device (for example, in a wireless or wired manner), and the power supply module 150 may receive electric energy input by the charging device, to store electricity by using a battery.

In some embodiments, still referring to FIG. 1, the wearable device 100 further includes a display 160. The display 160 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, a touch sensor may be disposed in the display to form a touchscreen. This is not limited in this embodiment of this application. It may be understood that, in some embodiments, the wearable device 100 may include the display 160, or may not include the display 160. For example, when the wearable device 100 is a band, the wearable device 100 may include a display or may not include a display. When the wearable device 100 is a watch, the wearable device 100 may include a display.

In some other embodiments, still referring to FIG. 1, the wearable device 100 may further include an audio device 170, and the audio device 170 may include a device that can receive or output a sound signal, such as a microphone, a loudspeaker, or a receiver.

The loudspeaker, also referred to as a "speaker", is configured to convert an audio electrical signal into a sound signal. The wearable device 100 may listen to music or answer a hands-free call by using the loudspeaker.

The receiver, also referred to as a "receiver", is configured to convert an audio electrical signal into a sound signal. When a call is answered or voice information is listened to by using the wearable device 100, voice may be listened to by placing the receiver close to a human ear.

A microphone, also referred to as a "mike" or a "mic", is configured to convert a sound signal into an electrical signal. When making a call or sending voice information, a user may make a sound near the microphone through the mouth of the user, to input a sound signal to the microphone. At least one microphone may be disposed in the wearable device 100. In some other embodiments, two microphones may be disposed in the wearable device 100, to collect a sound signal and further implement a noise reduction function. In some other embodiments, three, four, or more microphones may alternatively be disposed in the wearable device 100, to collect a sound signal, reduce noise, identify a sound source, implement a directional recording function, and the like.

In addition, the wearable device 100 may have a wireless communication function. In some embodiments, still referring to FIG. 1, the wearable device 100 may further include a wireless communication module 181, a mobile communication module 182, one or more antennas 1, and one or more antennas 2. The wearable device 100 may implement a wireless communication function by using the antenna 1, the antenna 2, the wireless communication module 181, and the mobile communication module 182.

In some embodiments, the wireless communication module 181 may provide a wireless communication solution that is applied to the wearable device 100 and that complies with various network communication protocols or communication technologies. For example, the network communication protocol may include a communication protocol such as a wireless local area network (wireless local area networks, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field wireless communication technology (near field communication, NFC), and an infrared technology (infrared, IR). For example, the wearable device 100 may establish a Bluetooth connection to another electronic device such as a mobile phone by using a Bluetooth protocol. In some other embodiments, the wireless communication module 181 may be one or more devices integrating at least one communication processing module.

The wireless communication module 181 receives an electromagnetic wave via the antenna 1, performs frequency modulation and filtering processing on an electromagnetic wave signal, and sends a processed signal to the processor 110. The wireless communication module 181 may further receive a to-be-sent signal from the processor 110, perform frequency modulation and amplification on the signal, convert the signal into an electromagnetic wave by using the antenna 1, and radiate the electromagnetic wave. In some embodiments, the wireless communication module 181 may be coupled to one or more antennas 1, so that the wearable device 100 may communicate with a network and another device by using a wireless communication technology.

In some embodiments, the mobile communication module 182 may provide a wireless communication solution that is applied to the wearable device 100 and that complies with various network communication protocols or communication technologies. For example, the network communication protocol may be various wired or wireless communication protocols, such as the Ethernet, global system for mobile communications (global system for mobile communications, GSM), general packet radio service (general packet radio service, GPRS), code division multiple access (code division multiple access, CDMA), wideband code division multiple access (wideband code division multiple access, WCDMA), time division code division multiple access (time-division code division multiple access, TD-SCDMA), long term evolution (long term evolution, LTE), voice over Internet protocol (voice over Internet protocol, VoIP), a communication protocol that supports a network slice architecture, or any other proper communication protocol. For example, the wearable device 100 may establish a wireless communication connection to another electronic device such as a mobile phone by using a WCDMA communication protocol.

In some other embodiments, the mobile communication module 182 may include at least one filter, a switch, a power amplifier, a low noise amplifier (low noise amplifier, LNA), and the like. In some other embodiments, at least some function modules of the mobile communication module 182 may be disposed in the processor 110. In some other embodiments, at least some function modules of the mobile communication module 182 and at least some modules of the processor 110 may be disposed in a same device.

The mobile communication module 182 may receive an electromagnetic wave via the antenna 2, perform processing such as filtering and amplification on the received electromagnetic wave, and transmit the electromagnetic wave to the modem processor for demodulation. The mobile communication module 182 may further amplify a signal obtained through modulation by the modem processor, convert the signal into an electromagnetic wave by using the antenna 2, and radiate the electromagnetic wave. In some embodiments, the mobile communication module 182 may be coupled to one or more antennas 2, so that the wearable device 100 may communicate with a network and another device by using a wireless communication technology.

In some embodiments, the wearable device further includes an electrode group 190, and the electrode group 190 includes at least two electrodes. The electrode group 190 may be disposed on one or more outer surfaces of the wearable device 100. The one or more processors 110 may monitor a voltage or a signal received by the electrode group 190. In some embodiments, the electrode may be configured to provide an ECG function for the wearable device 100. For example, when a user is in contact with a first electrode and a second electrode on the wearable device 100, the wearable device 100 may provide a two-lead ECG function, that is, the wearable device 100 may obtain an ECG signal based on a first electrical signal obtained through detection by the first electrode and a second electrical signal obtained through detection by the second electrode. For another example, when a user is in contact with a first electrode, a second electrode, and a third electrode on the wearable device 100, the wearable device 100 may provide a three-lead ECG function, that is, the wearable device 100 may obtain an ECG signal based on a first electrical signal obtained through detection by the first electrode, a second electrical signal obtained through detection by the second electrode, and a third electrical signal obtained through detection by the third electrode. Usually, more electrodes indicate that more electrical signals are collected and a more accurate ECG is obtained. The following embodiments of this application mainly use an example in which the wearable device 100 includes two electrodes.

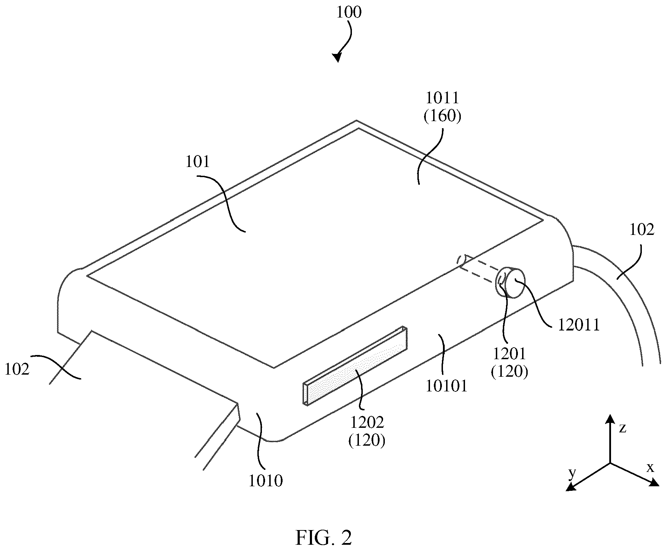

FIG. 2 is a schematic diagram of a structure of a wearable device 100 according to an embodiment of this application. In some embodiments, the wearable device 100 may be a smartwatch or a smart band. Referring to FIG. 2, the wearable device 100 includes a main body 101 and two wrist straps 102 (FIG. 2 shows a part of an area of the wrist strap 102). The wrist strap 102 may be fixedly connected or movably connected to the main body 101. The wrist strap 102 may be wound around a wrist, an arm, a leg, or another part of a body, to fasten the wearable device 100 to the body of a user. The main body 101 may include a housing 1010 and a cover 1011. The housing 1010 surrounds the cover 1011. For example, the housing 1010 includes a groove disposed at the top, the cover 1011 is accommodated in the groove, and an edge of the cover 1011 is adjacent to and is fastened to the groove of the housing 1010, to form a surface of the main body 101. A structure formed by the housing 1010 and the cover 1011 has an internal accommodating space, and may accommodate a combination of one or more components shown and not shown in FIG. 1, to implement various functions of the wearable device 100. The main body 101 further includes an input device 120. The accommodation space inside the structure formed by the cover 1011 and the housing 1010 may accommodate a part of the input device 120, and an exposed part of the input device 120 is easy to be in contact with the user.

As a surface of the main body 101, the cover 1011 may be used as a protection board of the main body 101, to avoid damage by preventing components accommodated in the housing 1010 from being exposed. For example, the cover 1011 may be transparent. For example, the cover 1011 may include a crystal, for example, a sapphire crystal, or the cover 1011 may be made of glass, plastic, or another material.

In some embodiments, the cover 1011 may be a display 160, and the user may interact with the wearable device 100 by using the display 160. For example, the display 160 may receive user input, and perform corresponding output in response to the user input. For example, the user may select to open or edit a graphic by touching or pressing a location of the graphic on the display 160 (or in another manner).

The input device 120 is attached to an outside of the housing 1010 and extends to an inside of the housing 1010. It may be understood that the rotatable input device 120 may be referred to as a button. In an embodiment in which the wearable device 100 is a watch, the rotatable input device 120 may be a crown of the watch. The input device 120 may be referred to as a crown.

The housing 1010 may be made of various materials, including but not limited to plastic, metal, alloy, and the like. The housing 1010 is provided with a mounting hole that cooperates with the input device 120, to accommodate a part of a structure of the input device 120.

It may be understood that the input device 120 is not limited to the structure shown in FIG. 2, and any mechanical component that can receive the user input may be used as the input device 120 in this embodiment of this application.

In some embodiments, referring to FIG. 2, the input device 120 of the wearable device 100 may be a button 1201, the button 1201 may be used as an example of the input device 120, and the button 1201 may be installed on a side surface 10101 of the housing 1010. In an embodiment in which the wearable device 100 is a watch, the button 1201 may be referred to as a crown.

In some other embodiments, still referring to FIG. 2, the input device 120 of the wearable device 100 may be a key 1202, and the key 1202 may be used as another example of the input device 120. The key 1202 may allow the user to press, so that the key 1202 moves, for example, pans or tilts, to implement movement input of the user. For example, the key 1202 may be installed on a side surface 10101 of the housing 1010, a part of the key 1202 is exposed, and the other part of the key 1202 extends from the side surface of the housing 1010 towards the inside of the housing 1010 (not shown in the figure). For example, the key 1202 may alternatively be disposed on a head 12011 of the button 1201, to implement movement input as well as rotation input. For example, the key 1202 may alternatively be disposed on a top surface on which the display 160 is mounted on the main body 101.

In some other embodiments, still referring to FIG. 2, the input device 120 may include a button 1201 and a key 1202. The button 1201 and the key 1202 may be disposed on a same surface of the housing 1010, for example, both disposed on a same side surface of the housing 1010. The button 1201 and the key 1202 may alternatively be disposed on different surfaces of the housing 1010. This is not limited in this embodiment of this application. It may be understood that the input device 120 may include one or more keys 1202, or may include one or more buttons 1201.

With development of wearable devices, the user has an increasingly high requirement on integrated functions of the wearable devices. For example, the wearable device may not only serve as a clock, but also detect a physiological parameter of the user. Generally, the user may measure a plurality of physiological parameters by using a physiological parameter sensor of the wearable device.

A type of the physiological parameter sensor and a type of the physiological parameter are not limited in this embodiment of this application.

For example, the physiological parameter sensor may include, but is not limited to, at least one of the following: an ACC sensor, a PPG sensor, and an ECG sensor.

For example, the physiological parameter may include atrial fibrillation, premature beat, a heart rate, arteriosclerosis, blood oxygen, sleep, stress, and the like.

When the user wants to measure a plurality of physiological parameters, the user needs to measure the plurality of physiological parameters by using a plurality of applications (Application, App) corresponding to the plurality of physiological parameters on the wearable device. An operation is complex, and user experience is poor.

Therefore, an embodiment of this application provides a measurement method 200, and the measurement method 200 is applied to the wearable device. According to the measurement method 200, the user may measure a plurality of physiological parameters at a time by using one APP, so that measurement efficiency is high, and user experience is relatively good.

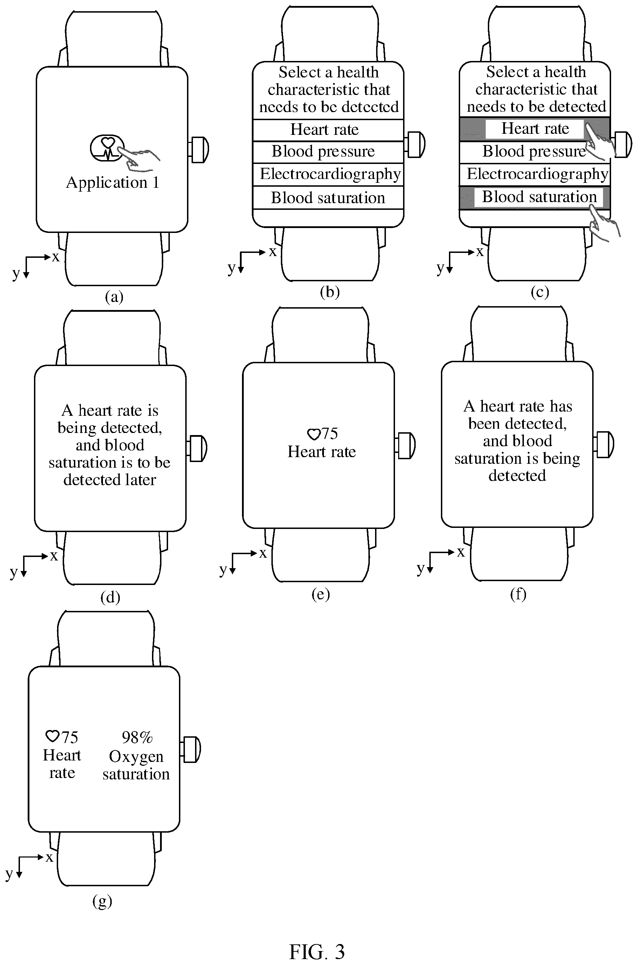

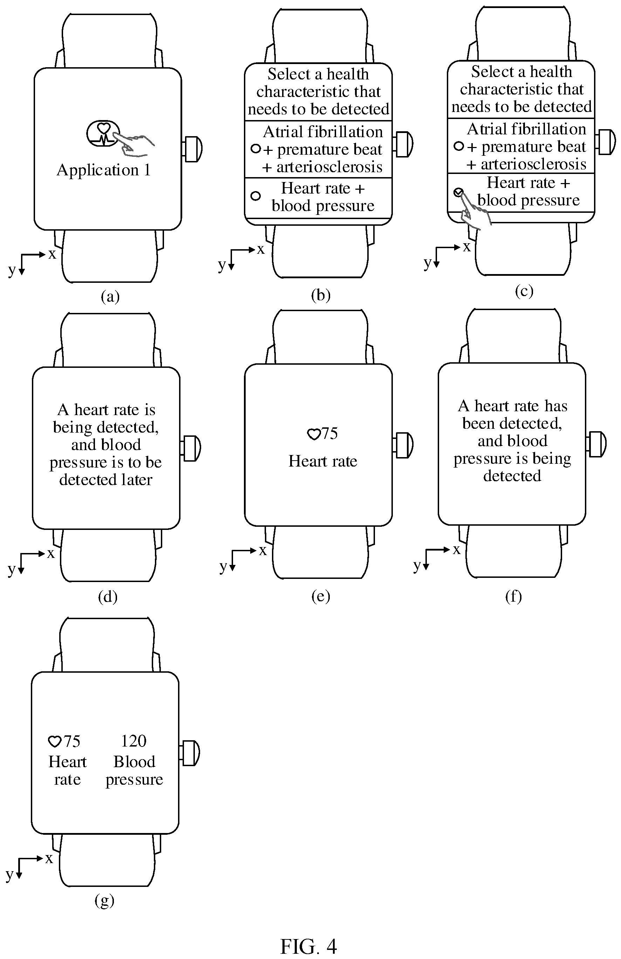

FIG. 3 and FIG. 4 are used as examples below to describe a schematic diagram of change of a graphical user interface (graphical user interface, GUI) of the wearable device in a process in which the user measures a plurality of physiological parameters by using one APP according to an embodiment of this application.

FIG. 3 is a schematic diagram of change of a GUI of a wearable device according to an embodiment of this application.

As shown in (a) in FIG. 3, an icon of an application 1 is displayed om a display interface of a wearable device 100.

For ease of description, in this embodiment of this application, an example in which the application 1 is a name of a physiological parameter measurement application is used for description. The name of the physiological parameter measurement application is not limited in this embodiment of this application.

In some embodiments, in addition to the icon of the application 1, icons of one or more other applications are further displayed on a display interface of the wearable device 100.

As shown in (a) in FIG. 3, after a user taps the icon of the application 1, a physiological parameter measurement interface may be displayed on the display interface of the wearable device 100. The physiological parameter measurement interface includes a plurality of physiological parameter identifiers.

In some embodiments, the plurality of physiological parameter identifiers may be displayed on the physiological parameter measurement interface in a separate manner.

For example, the plurality of physiological parameter identifiers may be displayed in a plurality of menu item options on the physiological parameter measurement interface.

A quantity of physiological parameters included in each menu option is not limited in this embodiment of this application.

For example, as shown in (b) in FIG. 3, each menu option includes an identifier of one physiological parameter. To be specific, a "heart rate" menu option, a "blood pressure" menu option, an "electrocardiography" menu option, and a "blood saturation" menu option are displayed on the display interface.

In some embodiments, the user may change, by performing an operation 1, the menu options displayed on the display interface.

A specific form of the operation 1 is not limited in this embodiment of this application.

For example, the operation 1 may be an operation of sliding up and down on the display interface by the user.

For example, the operation 1 may be an operation of rotating the input device 120 by the user.

The user selects, from the plurality of menu options, an identifier of a physiological parameter that needs to be measured.

In some embodiments, the user may select, from the plurality of menu options by performing an operation 2, a menu option corresponding to a physiological parameter that needs to be measured.

A specific form of the operation 2 is not limited in this embodiment of this application.

For example, the operation 2 may be an operation of touching and holding, by the user, a menu option corresponding to a physiological parameter that needs to be measured, for example, as shown in (c) in FIG. 3.

For example, the operation 2 may be a voice operation in which the user enters a physiological parameter that needs to be measured.

In this embodiment of this application, a specific presentation form, on the display interface of the wearable device, of a menu option that corresponds to a physiological parameter that needs to be measured and that is selected by the user is not limited.

For example, on the display interface of the wearable device, the menu option that corresponds to the physiological parameter that needs to be measured and that is selected by the user is highlighted. For example, as shown in (c) in FIG. 3, the "heart rate" menu option and the "blood saturation" menu option that are selected by the user are highlighted.

After the user selects the menu option corresponding to the physiological parameter that needs to be measured, the wearable device starts to measure the physiological parameter selected by the user.

In a possible implementation, after the user selects the menu option corresponding to the physiological parameter that needs to be measured, the wearable device may start, within a preset time period, to measure the physiological parameter selected by the user.

In another possible implementation, the physiological parameter measurement interface further includes a control used to start to measure the physiological parameter selected by the user. After the user selects the menu option corresponding to the physiological parameter that needs to be measured, the user may tap the control, so that the wearable device starts to measure the physiological parameter selected by the user.

(d) in FIG. 3 to (g) in FIG. 3 show change of a GUI of the wearable device in a process in which the wearable device starts to measure a heart rate and blood saturation that are selected by the user.

For example, the wearable device may measure, by using the following method 200, the heart rate and the blood saturation that are selected by the user. For a specific measurement process, refer to related descriptions in the following method 200. Details are not described herein.

In some embodiments, in a process of measuring the physiological parameter selected by the user, the wearable device may remind the user of measurement progresses of the physiological parameters of the user in ascending order of duration of the measured physiological parameters.

In this embodiment of this application, a form of reminding the user of the progress of measuring the physiological parameter of the user by the wearable device is not limited.

For example, the wearable device may remind, on the display interface of the wearable device, the user of the progress of measuring the physiological parameter of the user by the wearable device. For example, as shown in (d) in FIG. 3, the wearable device may display, on the display interface of the wearable device, reminder information of "a heart rate is being measured, and blood saturation is to be measured later". For example, as shown in (f) in FIG. 3, the wearable device may display, on the display interface of the wearable device, reminder information "a heart rate has been measured, and blood saturation is being measured".

In some embodiments, in a process in which the wearable device measures the physiological parameter selected by the user, the wearable device may remind the user to correctly wear the wearable device and/or correctly use a corresponding physiological parameter (for example, a heart rate and blood saturation) measurement function of the wearable device.

After completing measurement of the physiological parameter of the user, the wearable device outputs a measurement result corresponding to the measured physiological parameter.

In some embodiments, each time after completing measurement of one physiological parameter of the user, the wearable device may output a measurement result corresponding to the measured physiological parameter.

A manner of outputting the measured physiological parameter is not limited in this embodiment of this application.

For example, the wearable device displays the measured physiological parameter on the display interface of the wearable device.

In a possible implementation, each time after completing measurement of one physiological parameter of the user, the wearable device may output only a measurement result corresponding to the currently measured physiological parameter of the user.

In another possible implementation, each time after completing measurement of one physiological parameter of the user, the wearable device may not only output a measurement result corresponding to the currently measured physiological parameter of the user, but also output at least one of measurement results corresponding to measured physiological parameters of the user in current physiological parameter measurement. After completing measurement of all physiological parameters of the user, the wearable device finally outputs measurement results corresponding to all physiological parameters that need to be measured and that are selected by the user.

For example, as shown in (d) in FIG. 3 to (g) in FIG. 3, duration of measuring a heart rate is less than duration of measuring blood saturation. Therefore, the wearable device first measures the heart rate of the user, and then measures the blood saturation of the user. In other words, the wearable device first outputs a heart rate measurement result of the user, and then outputs a blood saturation measurement result of the user. As shown in (e) in FIG. 3, after measurement of the heart rate of the user is completed, the heart rate of the user is displayed on the display interface of the wearable device. As shown in (g) in FIG. 3, after measurement of the blood saturation of the user is completed, the heart rate of the user and the blood saturation of the user are displayed on the display interface of the wearable device.

In some other embodiments, after completing measurement of all physiological parameters of the user, the wearable device may output the measured physiological parameters.

A manner of outputting the measured physiological parameter is not limited in this embodiment of this application.

For example, the wearable device displays the measured physiological parameter on the display interface of the wearable device.

In this embodiment of this application, a quantity of finally output physiological parameter measurement results is not limited.

For example, the wearable device may finally output measurement results corresponding to all the physiological parameters that need to be measured and that are selected by the user.

For example, as shown in (g) in FIG. 3, the wearable device finally outputs a measurement result corresponding to the heart rate selected by the user and a measurement result corresponding to the blood saturation selected by the user.

FIG. 4 is a schematic diagram of change of a GUI of a wearable device according to another embodiment of this application.

Compared with FIG. 3, a difference lies in that physiological parameter identifiers in FIG. 4 may be displayed on a physiological parameter measurement interface in a combination manner.

For example, identifiers of a plurality of physiological parameters may be displayed in a menu option on the physiological parameter measurement interface in this manner.

For example, each menu option in FIG. 4 includes identifiers of a plurality of physiological parameters. For example, as shown in (b) in FIG. 4, a first menu option includes identifiers of three physiological parameters: "atrial fibrillation", "premature beat", and "arteriosclerosis". The second menu option includes identifiers of two physiological parameters: "heart rate" and "blood pressure".

In this embodiment of this application, a combination manner of the plurality of physiological parameters included in each menu option is not limited.

In some embodiments, the combination manner of the plurality of physiological parameters included in each menu option may be configured by a wearable device system.

In some other embodiments, the combination manner of the plurality of physiological parameters included in each menu option may be set by a user by using a corresponding setting option. The user may set, by using the setting option, a plurality of physiological parameters that the user wants to combine.

In addition, each menu option in FIG. 4 further includes a control used by the user to select the menu option. For example, as shown in (b) in FIG. 4, circular controls are displayed in an "atrial fibrillation + premature beat + arteriosclerosis" menu option and a "heart rate + blood pressure" menu option. In addition, in FIG. 4, on the display interface of the wearable device, the menu option selected by the user is specifically presented in a form that a control corresponding to a menu option selected by the user is displayed in a selected state. For example, as shown in (c) in FIG. 4, a circular control corresponding to the "heart rate + blood pressure + arteriosclerosis" menu option selected by the user is displayed in a form of "4".

For descriptions of another figure in FIG. 4, refer to descriptions of a corresponding figure in FIG. 3. Details are not described herein again.

In addition, for measurement of some physiological parameters, although types of collected data are the same, amounts of the collected data are different. For example, as shown in Table 1, for atrial fibrillation, premature beat, and arteriosclerosis, all types of collected data are PPG data and ECG data. However, for atrial fibrillation and premature beat, PPG data and ECG data need to be collected for 30 seconds. For arteriosclerosis, PPG data and ECG data need to be collected for 60 seconds. Therefore, for measurement of some physiological parameters, some collected data may be reused. However, because measurement of physiological parameters in an existing wearable device is independent of each other, when measuring a plurality of physiological parameters, the user does not share data. Consequently, measurement time is relatively long, and user experience is poor.

**Table 1**

| Physiological parameter | Type of collected data | Duration | Dependency |
|---|---|---|---|
| Arrhythmia characteristic (for example, atrial fibrillation, premature beat) | ACC data + PPG data + ECG data | 30 seconds | The user correctly wearing the device, and the user touching the electrode group, to implement ECG lead |
| Vascular characteristic (for example, arteriosclerosis) | ACC data + PPG data + ECG data | 60 seconds | The user correctly wearing the device, and the user touching the electrode group, to implement ECG lead |
| Blood oxygen characteristic | ACC data + PPG data | 20 seconds | The user correctly wearing the device |
| Stress characteristic | ACC data + PPG data | 60 seconds | The user correctly wearing the device |

Therefore, an embodiment of this application further provides a measurement method 300. The measurement method 300 is applied to a wearable device. According to the measurement method 300, data collected by a plurality of physiological parameter APPs can be reused, so that measurement efficiency is high, and user experience is relatively good.

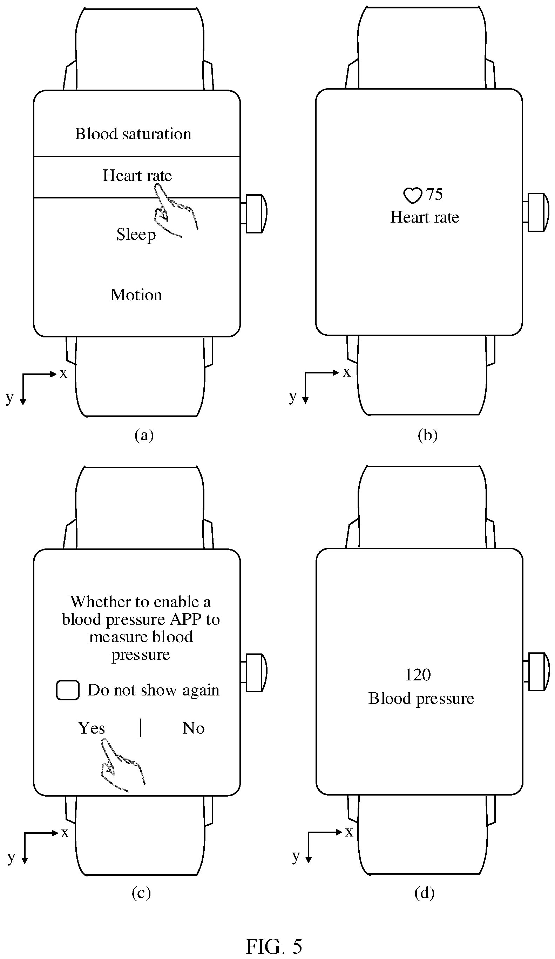

The following uses FIG. 5 as an example to describe a schematic diagram of change of a GUI of a wearable device in a process in which a user measures a plurality of physiological parameters by using a plurality of APPs according to an embodiment of this application.

FIG. 5 is a schematic diagram of change of a GUI of a wearable device according to still another embodiment of this application.

As shown in (a) in FIG. 5, a display interface of a wearable device 100 displays a "blood saturation" menu option, a "heart rate" menu option, a "sleep" menu option, and a "motion" menu option.

As shown in (a) in FIG. 5, after the user taps the "heart rate" menu option, the wearable device starts to measure a heart rate of the user.

For example, the wearable device may measure, by using the following method 300, the heart rate selected by the user. For a specific measurement process, refer to related descriptions in the following method 300. Details are not described herein.

In some embodiments, in a process in which the wearable device measures the heart rate of the user, the wearable device may remind the user to correctly wear the wearable device and/or correctly use a heart rate measurement function of the wearable device.

After completing measurement of the heart rate of the user, the wearable device outputs a measurement result obtained by measuring the heart rate of the user.

For example, as shown in (b) in FIG. 5, the wearable device displays the measured heart rate of the user on the display interface.

In some embodiments, after completing measurement of the heart rate of the user, the wearable device further determines that in a current heart rate measurement process, collected data may be further used as data collected when blood pressure is measured, and the wearable device may further remind the user whether to enable a blood pressure APP.

For example, as shown in (c) in FIG. 5, the wearable device displays, on the display interface, "whether to enable a blood pressure APP".

As shown in (c) in FIG. 5, the user determines to enable the blood pressure APP.

After it is determined that the user enables the blood pressure APP, a heart rate APP sends the collected data to the blood pressure APP, and the blood pressure APP analyzes the received data to obtain blood pressure of the user, and outputs the blood pressure of the user, for example, as shown in (d) in FIG. 5.

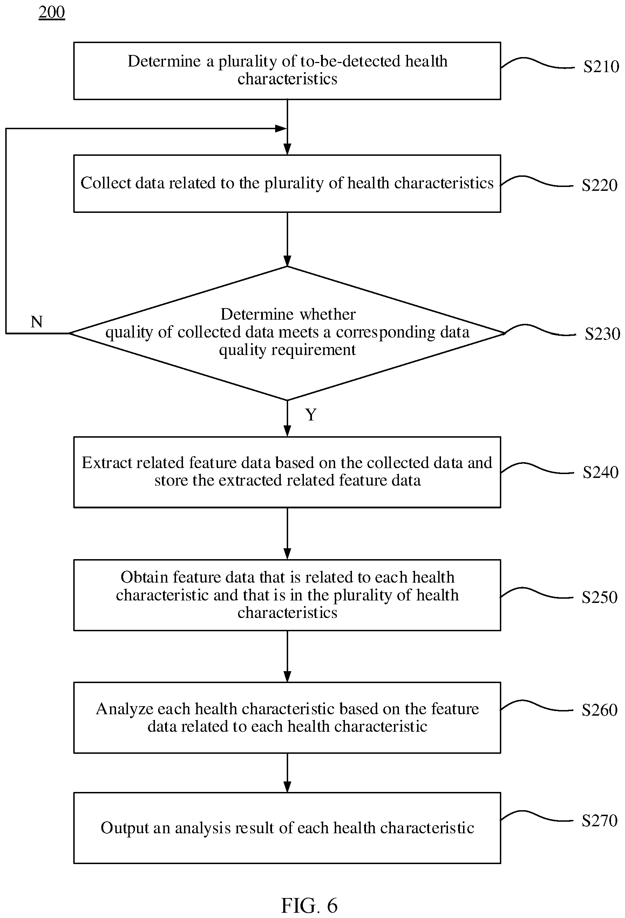

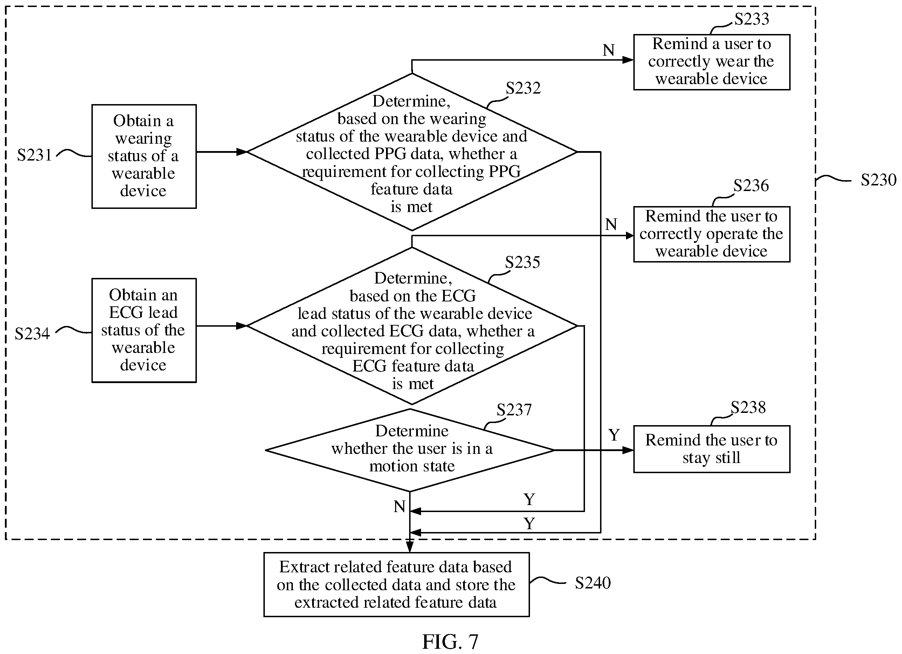

The foregoing describes, with reference to FIG. 3 to FIG. 5, the schematic diagram of change of a GUI of a wearable device in a process in which a user measures a plurality of physiological parameters according to an embodiment of this application. With reference to FIG. 6 and FIG. 7, the following describes a measurement method provided in an embodiment of this application.

With reference to FIG. 6, the following describes a physiological parameter measurement method 200 provided in an embodiment of this application. FIG. 6 is a schematic flowchart of a method 200 for measuring a physiological parameter according to an embodiment of this application. The method 200 includes the following steps.

S210: Determine a plurality of to-be-measured physiological parameters. A wearable device may determine the plurality of to-be-measured physiological parameters by receiving a physiological parameter measurement operation of the user.

In some embodiments, the wearable device may display a physiological parameter measurement interface on a display interface of the wearable device. The physiological parameter measurement interface includes a plurality of physiological parameter identifiers. The wearable device may determine the plurality of to-be-measured physiological parameters by using an operation of selecting at least two physiological parameter identifiers by the user.

In this embodiment of this application, a manner in which a plurality of physiological parameter identifiers are displayed on the physiological parameter measurement interface is not limited.

In a possible implementation, the plurality of physiological parameter identifiers may be displayed on the physiological parameter measurement interface in a separate manner.

For example, the plurality of physiological parameter identifiers are displayed in a plurality of menu item options on the physiological parameter measurement interface. For example, as shown in (b) in FIG. 3, one physiological parameter identifier is displayed in each menu item option on the physiological parameter measurement interface. In another possible implementation, the plurality of physiological parameter identifiers may be displayed on the physiological parameter measurement interface in a combination manner.

For example, on the physiological parameter measurement interface, identifiers of at least two physiological parameters are displayed in one menu option, and an identifier of at least one physiological parameter is included in another menu option.

For example, on the physiological parameter measurement interface, each menu option includes identifiers of at least two physiological parameters. For example, as shown in (b) in FIG. 4, two physiological parameter identifiers are displayed in each menu item option on the physiological parameter measurement interface.

In this embodiment of this application, specific physiological parameter identifiers that are displayed in a combination manner in the plurality of physiological parameter identifiers are not limited.

In an example, the physiological parameter identifiers that are displayed in a combination manner in the plurality of physiological parameter identifiers may be configured by a system. In another example, the physiological parameter identifiers that are displayed in a combination manner in the plurality of physiological parameter identifiers may be set by the user by using a corresponding setting option.

For example, a physiological parameter 1 and a physiological parameter 2 may be displayed in a combination manner. In a subsequent step, when data related to the physiological parameter 1 is extracted and data related to the physiological parameter 2 is extracted, some data is the same or all data is the same.

In an example, that when data related to the physiological parameter 1 is extracted and data related to the physiological parameter 2 is extracted, all data is the same may be understood as that a type of the extracted data related to the physiological parameter 1 is the same as a type of the extracted data related to the physiological parameter 2, and a data volume of the extracted data related to the physiological parameter 1 is the same as a data volume of the extracted data related to the physiological parameter 2.

For example, a type of data related to a physiological parameter may be an ACC data type, a PPG data type, and/or an ECG data type.

For example, as shown in Table 1, types of extracted data related to an arrhythmia characteristic (for example, atrial fibrillation and premature beat) are ACC data, PPG data, and ECG data, and a data volume required for extracting the data related to the arrhythmia characteristic is a data volume required for extracting the data related to the arrhythmia characteristic for 30 seconds. Types of extracted data related to a vascular characteristic are ACC data, PPG data, and ECG data, and a data volume required for extracting the data related to the vascular characteristic is a data volume required for extracting the data related to the vascular characteristic for 60 seconds. Types of extracted data related to a blood oxygen characteristic are ACC data and PPG data, and a data volume required for extracting the data related to the blood oxygen characteristic is a data volume required for extracting the data related to the blood oxygen characteristic for 20 seconds. Types of extracted data related to a stress characteristic are ACC data and PPG data, and a data volume required for extracting the data related to the stress characteristic is a data volume required for extracting the data related to the stress characteristic for 60 seconds.

For example, one of the physiological parameter 1 and the physiological parameter 2 may be atrial fibrillation, and the other may be premature beat.

In another example, that when data related to the physiological parameter 1 is extracted and data related to the physiological parameter 2 is extracted, some data is the same may be understood as that a type of the data related to the physiological parameter 1 is the same as a type of the data related to the physiological parameter 2, and a data volume of the data related to the physiological parameter 1 is different from a data volume of the data related to the physiological parameter 2.

For example, one of the physiological parameter 1 and the physiological parameter 2 may be an arrhythmia characteristic, and the other may be a blood oxygen characteristic. For another example, one of the physiological parameter 1 and the physiological parameter 2 may be a vascular characteristic, and the other may be an arrhythmia characteristic, a blood oxygen characteristic, or a stress characteristic. For another example, one of the physiological parameter 1 and the physiological parameter 2 may be a stress characteristic, and the other may be a blood oxygen characteristic.

In this embodiment of this application, an operation of how the user selects the at least two physiological parameter identifiers is not limited.

For example, if the user selects a first physiological parameter identifier and a second physiological parameter identifier from a plurality of physiological parameter identifiers displayed on the physiological parameter measurement interface displayed by the wearable device, the first physiological parameter identifier and the second physiological parameter identifier that are selected by the user are two to-be-measured physiological parameters. The first physiological parameter identifier and the second physiological parameter identifier are physiological parameter identifiers in the plurality of physiological parameter identifiers.

For ease of description, the following uses an example in which the user selects two physiological parameter identifiers for description.

In some embodiments, the user may select the plurality of to-be-measured physiological parameters by performing a plurality of operations.

For example, as shown in (c) in FIG. 3, the user separately taps a "heart rate" menu option and a "blood saturation" menu option, to select two to-be-measured physiological parameters, that is, a heart rate and blood saturation.

In some other embodiments, the user may select the plurality of to-be-measured physiological parameters by performing one operation. For example, as shown in (c) in FIG. 4, the user taps a "heart rate + blood pressure" menu option, to select two to-be-measured physiological parameters, that is, a heart rate and blood pressure.

After determining the plurality of to-be-measured physiological parameters, the wearable device may measure the plurality of physiological parameters.

In a possible implementation, after determining the plurality of to-be-measured physiological parameters, the wearable device starts, within a preset time period, to measure the plurality of physiological parameters selected by the user.

In another possible implementation, the physiological parameter measurement interface further includes a control used to start to measure the physiological parameter selected by the user. After the plurality of to-be-measured physiological parameters are determined, the user may tap the control, so that the wearable device starts to measure the physiological parameters selected by the user.

For a specific process in which the wearable device measures the plurality of physiological parameters, refer to S220 to S260.

In some embodiments, in a process in which the wearable device measures the physiological parameters selected by the user, the wearable device may remind the user to correctly wear the wearable device and/or correctly use a corresponding physiological parameter measurement function of the wearable device.

S220: Collect data related to the plurality of physiological parameters. In some embodiments, before the data related to the plurality of physiological parameters is collected, it may be first determined whether devices (for example, a physiological parameter sensor) related to the plurality of to-be-measured physiological parameters are in an on state. When there is a device in an off state in the devices related to the plurality of to-be-measured physiological parameters, the devices related to the plurality of to-be-measured physiological parameters are adjusted to an on state, to collect the data related to the plurality of physiological parameters. As shown in Table 1, for example, the plurality of physiological parameters include a physiological parameter related to the arrhythmia characteristic, and the wearable device needs to enable an ACC sensor, a PPG sensor, and an ECG sensor. For another example, the plurality of physiological parameters include the blood oxygen characteristic, and the wearable device needs to enable an ACC sensor and a PPG sensor. The ACC sensor collects magnitudes of accelerations of the wearable device in all directions. The PPG sensor collects an optical signal reflected by the skin of the user, and obtains a PPG signal based on the optical signal. The ECG sensor collects an electrical signal of each electrode in an electrode group disposed on the wearable device, and obtains an ECG signal based on the electrical signal. S230: Determine whether quality of the collected data meets a corresponding data quality requirement.

In an example, the wearable device may collect the data related to the plurality of physiological parameters by using a device that is of the wearable device and that is related to the plurality of to-be-measured physiological parameters.

In another example, the wearable device may be connected to another device and collect the data related to the plurality of physiological parameters by using a device that is of the another device and that is related to the plurality of to-be-measured physiological parameters. As shown in FIG. 7, S230 specifically includes S231 to S238.

Because the physiological parameters are different, types of data collected to measure the physiological parameters are also different. The following provides descriptions based on that the types of the collected data include a PPG data type and an ECG data type.

When the type of the collected data includes the PPG data type, S231 and S233 need to be performed. When the type of the collected data includes the ECG data type, S234 and S236 need to be performed.

S231: Obtain a wearing status of the wearable device.

For example, the wearing status of the wearable device includes whether the user wears the wearable device, whether the user correctly wears the wearable device, and the like.

For example, whether the user correctly wears the wearable device may be understood as whether a current manner in which the user wears the wearable device can well use a signal related to a physiological parameter.

S232: Determine, based on the wearing status of the wearable device and collected PPG data, whether a requirement for collecting PPG feature data is met.

When the wearing status of the wearable device is abnormal and/or the collected PPG data does not meet the requirement for collecting the PPG feature data, S233 is performed, and S220, S231, and S232 are cyclically performed until the wearing status of the wearable device is normal and/or the collected PPG data meets the requirement for collecting the PPG feature data. When the wearing status of the wearable device is normal and/or the collected PPG data meets the requirement for collecting the PPG feature data, S240 is performed.

For example, that the wearing status of the wearable device is abnormal may be understood as that the user does not wear the wearable device and/or the user does not correctly wear the wearable device.

For example, that the wearing status of the wearable device is normal may be understood as that the user wears the wearable device and/or the user correctly wears the wearable device.

For example, the requirement for collecting the PPG feature data includes that a waveform corresponding to the collected PPG data is smooth and has no burr, a spacing between a wave peak and a wave trough of the waveform corresponding to the collected PPG data meets a preset condition, and the like.

S233: Remind the user to correctly wear the wearable device.

In some embodiments, the wearable device may display reminder information on the display interface, to remind the user to correctly wear the wearable device. For example, the reminder information may be information "please wear the device with a finger away from the carpal bone".

Optionally, the wearable device may further display, on the display interface, a brief schematic diagram indicating that the user correctly wears the wearable device.

In some other embodiments, the wearable device may remind, by using a voice assistant, the user to correctly wear the wearable device.

S234: Obtain an ECG lead status of the wearable device.

For example, the ECG lead status of the wearable device includes that ECG of the wearable device is not in a lead state, and the ECG of the wearable device is in a lead state.

The ECG lead status of the wearable device may be determined based on whether a loop is formed between the electrode group and the user. When a loop is formed between the electrode group and the user, it is considered that the ECG of the wearable device is in the lead state. When no loop is formed between the electrode group and the user, it is considered that the ECG of the wearable device is not in the lead state.

S235: Determine, based on the ECG lead status of the wearable device and collected ECG data, whether a requirement for collecting ECG feature data is met.

When the ECG lead status of the wearable device is that the ECG of the wearable device is not in the lead state and/or the collected ECG data does not meet the requirement for collecting the ECG feature data, S236 is performed, and S220, S234, and S235 are cyclically performed until the ECG lead state of the wearable device is that the ECG of the wearable device is in the lead state and/or the collected ECG data meets the requirement for collecting the ECG feature data. When the ECG lead status of the wearable device is that the ECG of the wearable device is in the lead state and/or the collected ECG data meets the requirement for collecting the ECG feature data, S240 is performed.

For example, the requirement for collecting the ECG feature data includes that a waveform corresponding to the collected ECG data is smooth, has no burr, and the like.

S236: Remind the user to correctly operate the wearable device.

In some embodiments, the wearable device may display reminder information on the display interface, to remind the user to correctly operate the wearable device. For example, the reminder information may be information "please touch the ECG electrode with a finger".

Optionally, the wearable device may further display, on the display interface, a brief schematic diagram indicating that the user correctly operates the wearable device.

In some other embodiments, the wearable device may remind, by using a voice assistant, the user to correctly operate the wearable device.

S237 and S238 may be performed regardless of a type of the collected data.

S237: Determine, based on data collected by ACC, the data collected by the ACC.

For example, when there is a valid value in the data collected by the ACC, it may be considered that the user is in a motion state. When all the data collected by the ACC is invalid, it may be considered that the user is in a non-motion state.

For example, the valid value is a non-zero value, and the invalid value is 0.

When the user is in the motion state, S238 is performed, and S220 and S237 are cyclically performed until the user is in the non-motion state. When the user is in the non-motion state, S240 is performed.

S238: Remind the user to stay still.

In some embodiments, the wearable device may display reminder information on the display interface, to remind the user to stay still. For example, the reminder information may be information "please stay still".

In some other embodiments, the wearable device may remind, by using a voice assistant, the user to stay still.

When quality of the collected data meets a corresponding data quality requirement, S240 is performed. When the quality of the collected data does not meet the corresponding data quality requirement, S220 to S230 are cyclically performed until the quality of the collected data meets the corresponding data quality requirement.

S240: Extract related feature data based on the collected data, and store the extracted related feature data.

The feature data may be understood as feature data obtained by invoking a corresponding physiological parameter data algorithm.

For example, if the collected data includes data collected by using the ACC, the feature data corresponding to the data is extracted based on the data collected by the ACC.

For ease of description, the data collected by using the ACC is denoted as ACC data, and the feature data corresponding to the data collected by using the ACC is denoted as ACC feature data.

For example, if the collected data includes data collected by using the PPG sensor, the feature data corresponding to the data is extracted based on the data collected by using the PPG sensor.

For ease of description, the data collected by using the PPG sensor is denoted as PPG data, and the feature data corresponding to the data collected by using the PPG sensor is denoted as PPG feature data.

For example, if the collected data includes data collected by using the ECG sensor, the feature data corresponding to the data is extracted based on the data collected by using the ECG sensor.

For ease of description, the data collected by using the ECG sensor is denoted as ECG data, and the feature data corresponding to the data collected by using the ECG sensor is denoted as ECG feature data.

S250: Obtain feature data that is in the plurality of physiological parameters and that is related to each physiological parameter.

For example, as shown in FIG. 3, the user needs to measure two physiological parameters: a heart rate and blood saturation. In this case, in S250, feature data related to the heart rate and feature data related to the blood saturation need to be obtained.

The feature data related to the heart rate includes ACC feature data and PPG feature data, or the feature data related to the heart rate includes ACC feature data and ECG feature data. The feature data related to the blood saturation includes ACC feature data and PPG feature data.

For another example, as shown in FIG. 4, the user needs to measure two physiological parameters: a heart rate and blood pressure. In this case, in S250, feature data related to the heart rate and feature data related to the blood pressure need to be obtained.

The feature data related to the blood pressure includes ACC feature data and PPG feature data.

S260: Analyze each physiological parameter based on the feature data related to each physiological parameter.

The following uses two physiological parameters as an example to describe S260 in detail. For example,

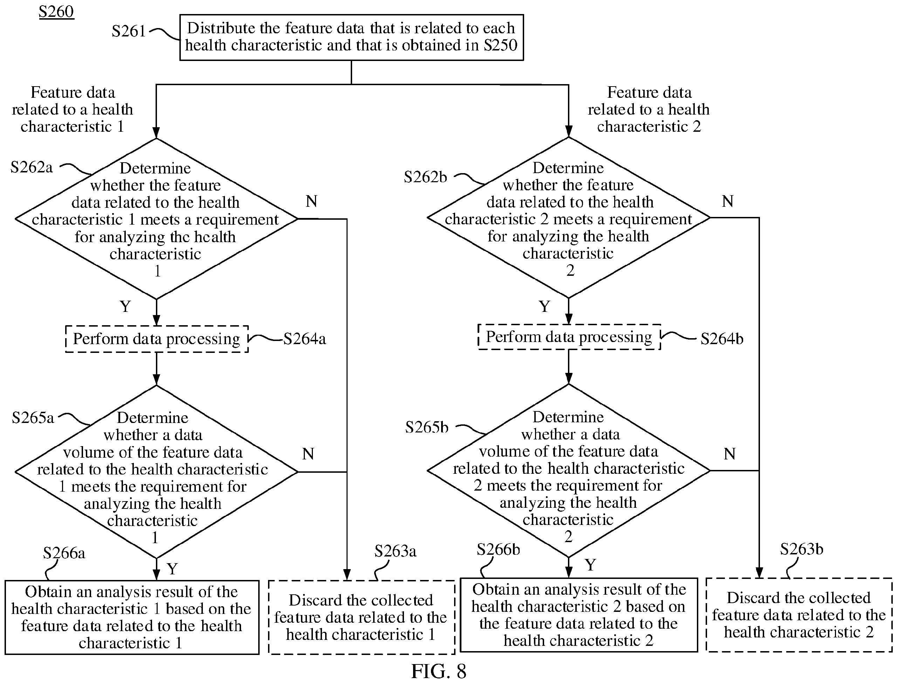

FIG. 8 is a schematic flowchart of analyzing two physiological parameters.

As shown in FIG. 8, S260 includes S261, S262a to S266a, and S262b to S266b.

S261: Extract the feature data that is related to each physiological parameter and that is obtained in S250.

Specifically, each physiological parameter is analyzed based on the feature data related to each physiological parameter. Processes of analyzing physiological parameters are independent of each other and do not interfere with each other.

After the feature data related to each physiological parameter is extracted, each physiological parameter needs to be analyzed. The following describes a specific procedure of analyzing each physiological parameter.

For example, S262a to S266a in FIG. 8 are a specific procedure of analyzing the physiological parameter 1 (an example of the first physiological parameter) and the physiological parameter 2 (an example of the second physiological parameter).

S262a: Determine, based on feature data that is related to the physiological parameter 1 and that is distributed in S261, whether the feature data related to the physiological parameter 1 meets a requirement for analyzing the physiological parameter 1.

It should be understood that for different physiological parameters, there are respective corresponding requirements for analyzing the physiological parameters.

For example, if the physiological parameter 1 is atrial fibrillation measurement, the requirement for analyzing the physiological parameter 1 may be a standard requirement of atrial fibrillation measurement. For example, a difference between a wave peak value and a wave trough value of a waveform corresponding to PPG data does not exceed a preset value.

When the feature data related to the physiological parameter 1 does not meet the requirement for analyzing the physiological parameter 1, the physiological parameter 1 cannot be analyzed.

In this case, in some embodiments, prompt information used to prompt that the physiological parameter cannot be obtained may be output, and/or S263a may be performed, that is, the collected feature data related to the physiological parameter 1 is discarded.

When the feature data related to the physiological parameter 1 meets the requirement for analyzing the physiological parameter 1, S264a is performed.

S264a: Perform data processing.

For example, data processing may be data restoration.

For example, if noise data exists in the collected feature data related to the physiological parameter 1, noise reduction processing may be performed on the collected feature data related to the physiological parameter 1, to restore the collected feature data related to the physiological parameter 1.

In some embodiments, S264a may be performed. In some other embodiments, S264a may not be performed.

S265a: Determine whether a data volume of the feature data related to the physiological parameter 1 meets a requirement on a data volume required for analyzing the physiological parameter 1.

It should be understood that for different physiological parameters, there are respective corresponding requirements on data volumes required for analyzing the physiological parameters.

As shown in Table 1, for example, for an arrhythmia characteristic, feature data related to the arrhythmia characteristic needs to be collected for 30 seconds. For another example, for a vascular physiological parameter, feature data related to the vascular physiological parameter needs to be collected for 60 seconds. For another example, for a blood oxygen characteristic, feature data related to the blood oxygen characteristic needs to be collected for 20 seconds. For another example, for a stress characteristic, feature data related to the stress characteristic needs to be collected for 60 seconds.

When the data volume of the feature data related to the physiological parameter 1 does not meet the requirement on the data volume required for analyzing the physiological parameter 1, the physiological parameter 1 cannot be analyzed.

In this case, in some embodiments, prompt information used to prompt that the physiological parameter cannot be obtained may be output, and/or S263a may be performed, that is, the collected feature data related to the physiological parameter 1 is discarded.

When the data volume of the feature data related to the physiological parameter 1 meets the requirement on the data volume required for analyzing the physiological parameter 1, S266a is performed.

S266a: Obtain an analysis result of the physiological parameter 1 based on the feature data related to the physiological parameter.

For example, the feature data related to the physiological parameter 1 is input into an analysis model of the physiological parameter 1, to obtain the analysis result of the physiological parameter 1.

It should be understood that for different physiological parameters, there are respective corresponding physiological parameter analysis models.

A specific procedure of analyzing the physiological parameter 2 is described above with reference to S262a to S266a. A specific procedure of analyzing the physiological parameter 2 is described below with reference to S262b to S266b in FIG. 8.

The specific procedure of analyzing the physiological parameter 2 is similar to the specific procedure of analyzing the physiological parameter 1. For related descriptions of S262b to S266b, refer to the foregoing descriptions of S262a to S266a. Details are not described herein again.

It should be understood that, in FIG. 8, two physiological parameters (the physiological parameter 1 and the physiological parameter 2) are used as an example to describe S260 in detail. In other words, in S261, two pieces of data related to the physiological parameters are distributed. For analysis of N (N is greater than 2) physiological parameters, in S261, N pieces of feature data related to the physiological parameters are distributed. In addition, the N physiological parameters need to be independently analyzed. To be specific, a procedure similar to S262a to S266a needs to be performed based on each of the N physiological parameters.

S270: Output an analysis result of each physiological parameter.

In some embodiments, each time after an analysis result of one physiological parameter is obtained, the analysis result of the physiological parameter may be output.

In some other embodiments, only after analysis results of a plurality of physiological parameters are obtained, the analysis results of the plurality of physiological parameters may be output.

An embodiment of this application provides another measurement method 300. The measurement method 300 is applied to a wearable device. According to the measurement method 300, after a user completes measurement of a physiological parameter by using an App, when data collected for the physiological parameter may be further used to analyze another physiological parameter, an App corresponding to the another physiological parameter may be further invoked to output an analysis result of the another physiological parameter. Data collected by a plurality of physiological parameter Apps can be reused, so that measurement efficiency is high and user experience is relatively good.

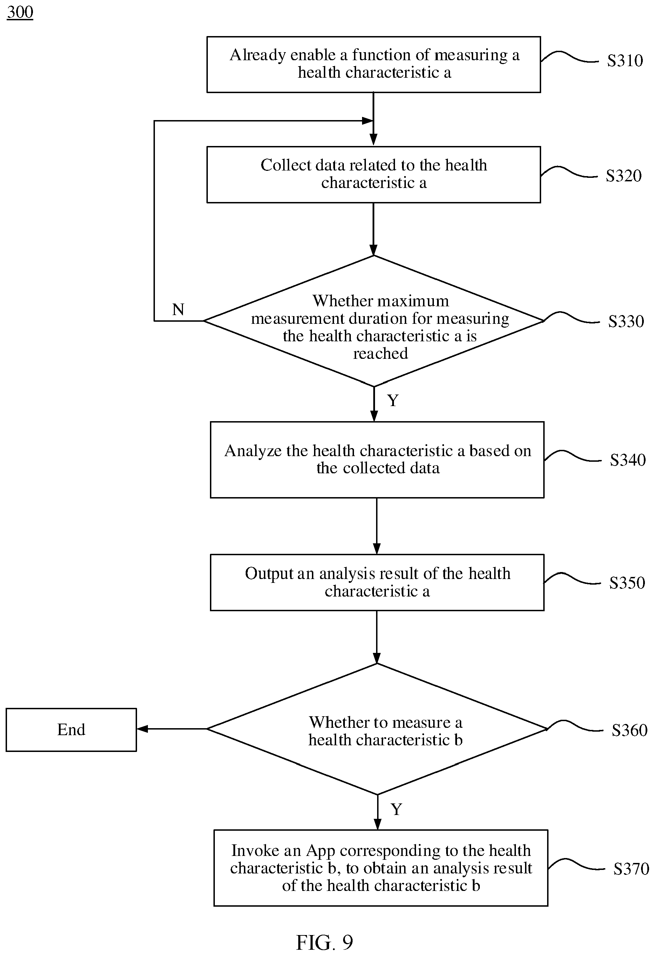

With reference to FIG. 6 to FIG. 9, the foregoing describes the measurement method 200 provided in an embodiment of this application. With reference to FIG. 9, the following describes the measurement method 300 provided in an embodiment of this application. FIG. 9 is a schematic flowchart of a measurement method 300 according to an embodiment of this application. The method 300 includes the following steps.

S310: Already enable a function of measuring a physiological parameter a (another example of a first physiological parameter).

A manner of enabling the function of measuring the physiological parameter a is not limited in this application.

S320: Collect data related to the physiological parameter a.

As shown in Table 1, for example, data related to an arrhythmia characteristic includes ACC data, PPG data, and ECG data. For another example, data related to a vascular physiological parameter includes ACC data, PPG data, and ECG data. For another example, data related to a blood oxygen characteristic includes ACC data and PPG data. For another example, data related to a stress characteristic includes ACC data and PPG data.

S330: Determine whether maximum measurement duration for measuring the physiological parameter a is reached.

Each physiological parameter corresponds to one piece of maximum measurement duration.

As shown in Table 1, for example, maximum measurement duration corresponding to the arrhythmia characteristic is 30 seconds. For another example, maximum measurement duration corresponding to the vascular physiological parameter and maximum measurement duration corresponding to the stress characteristic are both 60 seconds. For another example, maximum measurement duration corresponding to the blood oxygen characteristic is 20 seconds.

When the maximum measurement duration for measuring the physiological parameter a is not reached, S320 and S330 are repeatedly performed until the maximum measurement duration for measuring the physiological parameter a is reached.

When the maximum measurement duration for measuring the physiological parameter a is reached, S340 is performed.

S340: Analyze the physiological parameter a based on the collected data.

For example, the collected data is input into an analysis model of the physiological parameter a, to obtain an analysis result of the physiological parameter a.

It should be understood that for different physiological parameters, there are respective corresponding physiological parameter analysis models.

S350: Output the analysis result of the physiological parameter a.

In some embodiments, the analysis result of the physiological parameter a may be output by using a display interface of an electronic device.

For example, as shown in (b) in FIG. 5, a heart rate value is displayed on the display interface of the electronic device.

The physiological parameter a is measured by using S310 to S350.

In this case, if an analysis result of the physiological parameter b may be further obtained based on the data that is related to the physiological parameter a and that is collected in S320, S360 may be further performed.

That an analysis result of the physiological parameter b (another example of a second physiological parameter) may be further obtained based on the data that is related to the physiological parameter a and that is collected in S320 may be understood that the data that is related to the physiological parameter a and that is collected in S320 is also data that needs to be collected when the physiological parameter b is analyzed. In other words, a data volume of the data that is related to the physiological parameter a and that is collected in S320 meets a data volume of data that needs to be collected when the physiological parameter b is analyzed, and a data type of the data that is related to the physiological parameter a and that is collected in S320 includes a type of data that needs to be collected when the physiological parameter b is analyzed.

As shown in Table 1, for example, the physiological parameter a may be a physiological parameter related to the arrhythmia characteristic, and the physiological parameter b may be a physiological parameter related to the blood oxygen characteristic. For another example, the physiological parameter a may be a physiological parameter related to the vascular physiological parameter, and the physiological parameter b may be a physiological parameter related to the arrhythmia characteristic, a physiological parameter related to the blood oxygen characteristic, or a physiological parameter related to the stress characteristic. For another example, the physiological parameter a may be a physiological parameter related to the stress characteristic, and the physiological parameter b may be a physiological parameter related to the blood oxygen characteristic.

S360: Determine whether to measure the physiological parameter b.

In some embodiments, prompt information used to prompt whether to measure the physiological parameter b may be output.

In a possible implementation, the prompt information used to prompt whether to measure the physiological parameter b may be displayed on the display interface of the electronic device.

For example, when collected data related to a heart rate (an example of the physiological parameter a) may further be used to analyze blood pressure (an example of the physiological parameter b), as shown in (c) in FIG. 5, prompt information "whether to enable a blood pressure APP to measure blood pressure" is displayed on a display interface of a watch.

In some embodiments, whether to measure the physiological parameter b is determined based on an operation of the user.

In a possible implementation, a control used to measure the physiological parameter b and/or a control used to not measure the physiological parameter b may be displayed on the display interface of the electronic device. It is determined, based on an operation of tapping a corresponding control by the user, whether the physiological parameter b needs to be measured.

For example, as shown in (c) in FIG. 5, in addition to the prompt information "whether to enable a blood pressure APP to measure blood pressure", a "yes" control and a "no" control are further displayed on the display interface of the watch. It is determined, based on an operation of selecting the "yes" control by the user, that the physiological parameter b needs to be measured. It is determined, based on an operation of selecting the "no" control by the user, that the physiological parameter b does not need to be measured.

S310 to S360 are performed by a corresponding App for measuring the physiological parameter a.

When the physiological parameter b does not need to be measured, S370 does not need to be performed.

When the physiological parameter b needs to be measured, S370 is performed.

S370: Invoke an App corresponding to the physiological parameter b to measure the physiological parameter b.

For example, the corresponding App for measuring the physiological parameter a sends a measurement message to a corresponding App for measuring the physiological parameter b. The measurement message includes data that is related to the physiological parameter a and that is collected by the corresponding App for measuring the physiological parameter a, and the measurement message is used to request an analysis result of the physiological parameter b. The corresponding App for measuring the physiological parameter b may obtain the analysis result of the physiological parameter b based on the measurement message. For example, the corresponding App for measuring the physiological parameter b inputs, into an analysis model of the physiological parameter b, the data that is related to the physiological parameter a, that is collected by the corresponding App for measuring the physiological parameter a, and that is included in the measurement message, to obtain the analysis result of the physiological parameter b.

Optionally, after receiving the measurement message, the corresponding App for measuring the physiological parameter b may further process the data in the measurement message, and then obtain the analysis result of the physiological parameter b based on processed data.

S3 50: Output the analysis result of the physiological parameter b.

In some embodiments, the corresponding App for measuring the physiological parameter b may output the analysis result of the physiological parameter b by using the display interface of the electronic device.

For example, as shown in (d) in FIG. 5, a blood pressure value is displayed on the display interface of the electronic device.

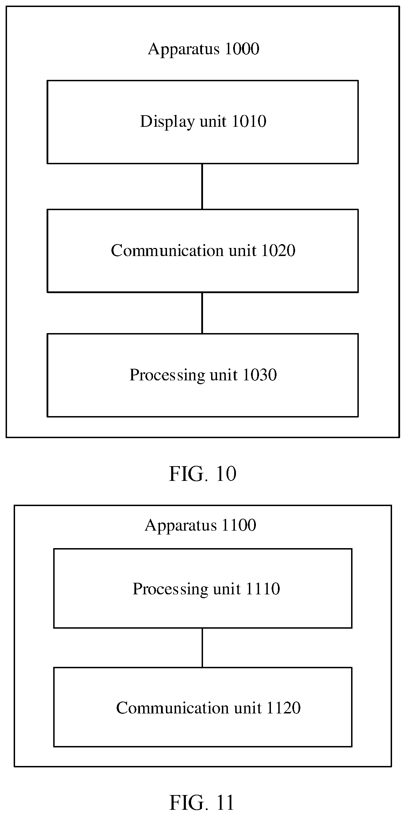

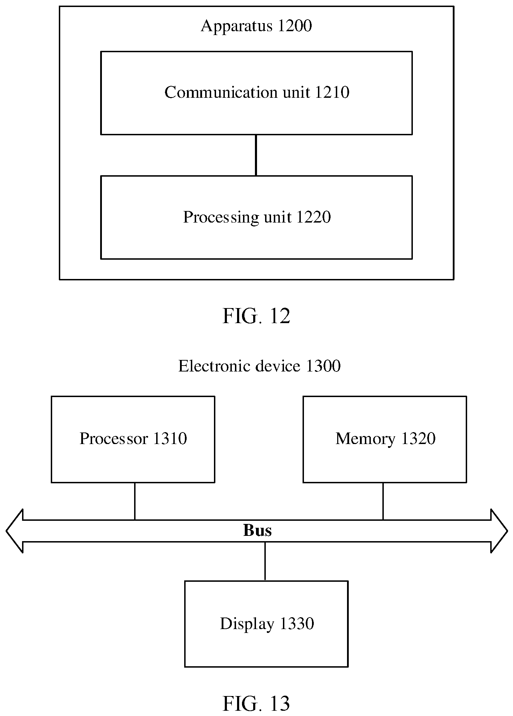

With reference to FIG. 3 to FIG. 9, the foregoing describes in detail the method for measuring a physiological parameter provided in an embodiment of this application. The following describes apparatus embodiments of this application in detail with reference to FIG. 10 and FIG. 13. It should be understood that the descriptions of the method embodiments correspond to descriptions of the apparatus embodiments. Therefore, for parts that are not described in detail, refer to the descriptions in the foregoing method embodiments.

FIG. 10 is a schematic block diagram of an apparatus according to an embodiment of this application. It should be understood that the apparatus 1000 may perform the method 200 shown in FIG. 6 to FIG. 8. The apparatus 1000 includes:
a display unit 1010, configured to display a physiological parameter measurement interface, where the physiological parameter measurement interface includes a plurality of physiological parameter identifiers;
a communication unit 1020, configured to receive a physiological parameter measurement operation, where the physiological parameter measurement operation includes an operation of selecting a first physiological parameter identifier and a second physiological parameter identifier, and the first physiological parameter identifier and the second physiological parameter identifier are physiological parameter identifiers in the plurality of physiological parameter identifiers; and
a processing unit 1030, configured to measure a first physiological parameter and a second physiological parameter of a measured object by using a physiological parameter sensor based on the physiological parameter measurement operation.

Optionally, the processing unit 1030 is further specifically configured to: collect data of the measured object by using the physiological parameter sensor based on the physiological parameter measurement operation; extract data related to the first physiological parameter and data related to the second physiological parameter from the collected data; obtain the first physiological parameter based on the data related to the first physiological parameter; and obtain the second physiological parameter based on the data related to the second physiological parameter.

Optionally, the processing unit 1030 is further configured to: determine whether data meets a data quality requirement; and if the data does not meet the data quality requirement, re-collect data of the measured object by using the physiological parameter sensor, and update the data with the re-collected data until the data meets the data quality requirement.

Optionally, the processing unit 1030 is further specifically configured to: extract, based on the data related to the first physiological parameter, feature data related to the first physiological parameter; determine whether the feature data of the first physiological parameter meets a requirement for analyzing the first physiological parameter; and obtain the first physiological parameter based on the feature data related to the first physiological parameter when the feature data of the first physiological parameter meets the requirement for analyzing the first physiological parameter.

Optionally, the processing unit 1030 is further specifically configured to: determine whether the feature data of the first physiological parameter meets the requirement for analyzing the first physiological parameter; determine whether a data volume corresponding to the feature data of the first physiological parameter meets the requirement for analyzing the first physiological parameter when the feature data of the first physiological parameter meets the requirement for analyzing the first physiological parameter; and obtain the first physiological parameter based on the feature data related to the first physiological parameter when the data volume corresponding to the feature data of the first physiological parameter meets the requirement for analyzing the first physiological parameter.

Optionally, the processing unit 1030 is further specifically configured to: perform data processing on the feature data of the first physiological parameter; and determine whether the data volume corresponding to the feature data of the first physiological parameter meets the requirement for analyzing the first physiological parameter when processed feature data of the first physiological parameter meets the requirement for analyzing the first physiological parameter.

Optionally, the processing unit 1030 is further specifically configured to: extract, based on the data related to the second physiological parameter, feature data related to the second physiological parameter; determine whether the feature data of the second physiological parameter meets a requirement for analyzing the second physiological parameter; and obtain the second physiological parameter based on the feature data related to the second physiological parameter when the feature data of the second physiological parameter meets the requirement for analyzing the second physiological parameter.

Optionally, the processing unit 1030 is further specifically configured to: determine whether the feature data of the second physiological parameter meets the requirement for analyzing the second physiological parameter; determine whether a data volume corresponding to the feature data of the second physiological parameter meets the requirement for analyzing the second physiological parameter when the feature data of the second physiological parameter meets the requirement for analyzing the second physiological parameter; and obtain the second physiological parameter based on the feature data related to the second physiological parameter when the data volume corresponding to the feature data of the second physiological parameter meets the requirement for analyzing the second physiological parameter.

Optionally, the processing unit 1030 is further specifically configured to: perform data processing on the feature data of the second physiological parameter; and determine whether the data volume corresponding to the feature data of the second physiological parameter meets the requirement for analyzing the second physiological parameter when processed feature data of the second physiological parameter meets the requirement for analyzing the second physiological parameter.

Optionally, the display unit 1010 is further configured to display the first physiological parameter and the second physiological parameter of the measured object.

Optionally, the display unit 1010 is further specifically configured to: display the first physiological parameter after measurement of the first physiological parameter is completed; and display the second physiological parameter after measurement of the second physiological parameter is completed.

Optionally, the display unit 1010 is further specifically configured to display the first physiological parameter and the second physiological parameter after measurement of the first physiological parameter and the second physiological parameter is completed.

Optionally, the physiological parameter sensor includes at least one of the following: an ACC sensor, a PPG sensor, and an ECG sensor.

Optionally, the physiological parameter includes at least one of the following: atrial fibrillation, premature beat, a heart rate, arteriosclerosis, blood oxygen, sleep, and stress.

Optionally, the extracted data related to the first physiological parameter is partially or completely the same as the extracted data related to the second physiological parameter.

FIG. 11 is a schematic block diagram of an apparatus according to an embodiment of this application. It should be understood that the apparatus 1100 may perform the method 300 shown in FIG. 9. The apparatus 1100 includes:
a processing unit 1110, configured to collect, by using a physiological parameter sensor, data that is related to a first physiological parameter and that is of a measured object, where
the processing unit 1110 is further configured to obtain the first physiological parameter based on the data related to the first physiological parameter; and
the processing unit 1110 is further configured to detect an operation of measuring a second physiological parameter, where the second physiological parameter is different from the first physiological parameter; and
a communication unit 1120, configured to send a measurement message to a second application in response to the operation, where the measurement message includes the data related to the first physiological parameter.

Optionally, after the processing unit 1110 is further configured to obtain the first physiological parameter based on the data related to the first physiological parameter, the processing unit 1110 is further configured to determine the second physiological parameter based on the data related to the first physiological parameter.

FIG. 12 is a schematic block diagram of an apparatus according to an embodiment of this application. It should be understood that the apparatus 1200 may perform the method 300 shown in FIG. 9. The apparatus 1200 includes:
a communication unit 1210, configured to receive a measurement message from a first application, where the measurement message includes data related to a first physiological parameter; and
a processing unit 1220, configured to obtain a second physiological parameter based on the data related to the first physiological parameter.

FIG. 13 is a schematic diagram of a hardware structure of a wearable device according to an embodiment of this application. An electronic device 1100 shown in FIG. 13 includes one or more memories 1110, one or more processors 1120, and a display 1130.

The one or more memories 1110 store one or more computer programs, and the one or more computer programs include instructions.

In a possible implementation, when the instructions are by the one or more processors 1120, the electronic device 1100 is enabled to perform the method 200 or the method 300 in the foregoing embodiments.

In another possible implementation, the processor 1120 includes a central processing unit (Central Processing Unit, CPU) and a neural network processing unit (Neural-network Processing Unit, NPU). When the instructions are run by the NPU, the electronic device 1100 is enabled to perform the method 200 or the method 300 in the foregoing embodiments. For example, when the instructions are run by the NPU, the NPU may perform a solution of obtaining the first physiological parameter based on the data related to the first physiological parameter in the method 200.

The display 1230 is configured to display information. For example, the display 1230 is configured to display the first physiological parameter, the second physiological parameter, and the like in the foregoing embodiments.

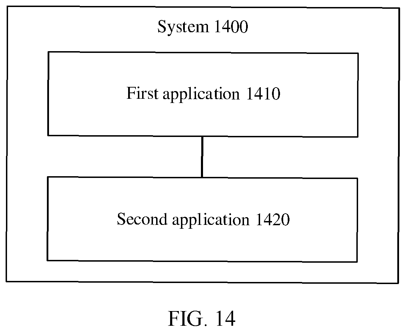

An embodiment of this application further provides a system. FIG. 14 is a schematic block diagram of a system 1400 according to an embodiment of this application. As shown in FIG. 14, the system 1400 includes:
a first application 1410, used to collect data that is related to a first physiological parameter and that is of a measured object, where
the first application 1410 is further configured to obtain the first physiological parameter based on the data related to the first physiological parameter;
the first application 1410 is further configured to detect an operation of measuring a second physiological parameter, where the second physiological parameter is different from the first physiological parameter; and
the first application 1410 is further configured to send a measurement message to a second application in response to the operation, where the measurement message includes the data related to the first physiological parameter; and
the second application 1420, configured to obtain the second physiological parameter based on the data related to the first physiological parameter.

Optionally, after the first application 1410 is further configured to obtain the first physiological parameter based on the data related to the first physiological parameter, the first application 1410 is further configured to determine the second physiological parameter based on the data related to the first physiological parameter.

An embodiment of this application further provides a chip. The chip includes a transceiver unit and a processing unit. The transceiver unit may be an input/output circuit or a communication interface. The processing unit is a processor, a microprocessor, or an integrated circuit integrated on the chip. The chip may perform the method in the foregoing method embodiments.

An embodiment of this application further provides a computer readable storage medium. The computer readable storage medium stores instructions. When the instructions are executed, the method in the foregoing method embodiments is performed.

An embodiment of this application further provides a computer program product including instructions. When the instructions are executed, the method in the foregoing method embodiments is performed.

It should also be understood that in this embodiment of this application, the memory may include a read-only memory and a random access memory, and provide instructions and data to the processor. Apart of the processor may further include a non-volatile random access memory. For example, the processor may further store information of a device type.

It should be understood that sequence numbers of the foregoing processes do not mean execution sequences in various embodiments of this application. The execution sequences of the processes should be determined according to functions and internal logic of the processes, and should not be construed as any limitation on the implementation processes of embodiments of this application.

A person of ordinary skill in the art may be aware that, in combination with the examples described in the embodiments disclosed in this specification, units and algorithm steps may be implemented by electronic hardware or a combination of computer software and electronic hardware. Whether the functions are performed by hardware or software depends on particular applications and design constraint conditions of the technical solutions. A person skilled in the art may use a different method to implement the described functions for each particular application, but it should not be considered that the implementation goes beyond the scope of this application.

It may be clearly understood by a person skilled in the art that, for the purpose of convenient and brief description, for a detailed working process of the foregoing system, apparatus, and unit, refer to a corresponding process in the foregoing method embodiments. Details are not described herein again.

In the several embodiments provided in this application, it should be understood that the disclosed system, apparatus, and method may be implemented in other manners. For example, the described apparatus embodiment is merely an example. For example, division into the units is merely logical function division and may be another division manner in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In addition, functional units in the embodiments of this application may be integrated into one processing unit, each of the units may exist alone physically, or two or more units are integrated into one unit.

When the functions are implemented in the form of a software functional unit and sold or used as an independent product, the functions may be stored in a computer readable storage medium. Based on such an understanding, the technical solutions of this application essentially, or the part contributing to the prior art, or some of the technical solutions may be implemented in a form of a software product. The software product is stored in a storage medium, and includes several instructions for instructing a computer device (which may be a personal computer, a server, or a network device) to perform all or some of the steps of the methods described in the embodiments of this application. The foregoing storage medium includes any medium that can store program code, such as a USB flash drive, a removable hard disk, a read-only memory (read-only memory, ROM), a random access memory (random access memory, RAM), a magnetic disk, or an optical disc.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A method for measuring a physiological parameter, wherein the method is applied to an electronic device comprising a physiological parameter sensor, and the method comprises:
displaying a physiological parameter measurement interface, wherein the physiological parameter measurement interface comprises a plurality of physiological parameter identifiers;
receiving a physiological parameter measurement operation, wherein the physiological parameter measurement operation comprises an operation of selecting a first physiological parameter identifier and a second physiological parameter identifier, and the first physiological parameter identifier and the second physiological parameter identifier are physiological parameter identifiers in the plurality of physiological parameter identifiers; and
measuring a first physiological parameter and a second physiological parameter of a measured object by using the physiological parameter sensor based on the physiological parameter measurement operation.

2. The method according to claim 1, wherein the measuring a first physiological parameter and a second physiological parameter of a measured object by using the physiological parameter sensor in response to the physiological parameter measurement operation comprises:
collecting data of the measured object by using the physiological parameter sensor based on the physiological parameter measurement operation;
extracting data related to the first physiological parameter and data related to the second physiological parameter from the collected data;
obtaining the first physiological parameter based on the data related to the first physiological parameter; and
obtaining the second physiological parameter based on the data related to the second physiological parameter.

3. The method according to claim 2, wherein after the collecting data of the measured object by using the physiological parameter sensor based on the physiological parameter measurement operation, the method further comprises:
determining whether the data meets a data quality requirement; and
when the data does not meet the data quality requirement, re-collecting data of the measured object by using the physiological parameter sensor, and updating the data with the re-collected data until the data meets the data quality requirement.

4. The method according to claim 2 or 3, wherein the obtaining the first physiological parameter based on the data related to the first physiological parameter comprises:
extracting, based on the data related to the first physiological parameter, feature data related to the first physiological parameter;
determining whether the feature data of the first physiological parameter meets a requirement for analyzing the first physiological parameter; and
obtaining the first physiological parameter based on the feature data related to the first physiological parameter when the feature data of the first physiological parameter meets the requirement for analyzing the first physiological parameter.

5. The method according to claim 4, wherein the determining whether the feature data of the first physiological parameter meets a requirement for analyzing the first physiological parameter comprises:
determining whether the feature data of the first physiological parameter meets the requirement for analyzing the first physiological parameter; and
determining whether a data volume corresponding to the feature data of the first physiological parameter meets the requirement for analyzing the first physiological parameter when the feature data of the first physiological parameter meets the requirement for analyzing the first physiological parameter; and
the obtaining the first physiological parameter based on the feature data related to the first physiological parameter when the feature data of the first physiological parameter meets the requirement for analyzing the first physiological parameter comprises:
obtaining the first physiological parameter based on the feature data related to the first physiological parameter when the data volume corresponding to the feature data of the first physiological parameter meets the requirement for analyzing the first physiological parameter.

6. The method according to claim 5, wherein after the feature data of the first physiological parameter meets the requirement for analyzing the first physiological parameter, the method further comprises:
performing data processing on the feature data of the first physiological parameter; and
the determining whether a data volume corresponding to the feature data of the first physiological parameter meets the requirement for analyzing the first physiological parameter when the feature data of the first physiological parameter meets the requirement for analyzing the first physiological parameter comprises:
determining whether the data volume corresponding to the feature data of the first physiological parameter meets the requirement for analyzing the first physiological parameter when processed feature data of the first physiological parameter meets the requirement for analyzing the first physiological parameter.

7. The method according to any one of claims 2 to 6, wherein the obtaining the second physiological parameter based on the data related to the second physiological parameter comprises:
extracting, based on the data related to the second physiological parameter, feature data related to the second physiological parameter;
determining whether the feature data of the second physiological parameter meets a requirement for analyzing the second physiological parameter; and
obtaining the second physiological parameter based on the feature data related to the second physiological parameter when the feature data of the second physiological parameter meets the requirement for analyzing the second physiological parameter.

8. The method according to claim 7, wherein the determining whether the feature data of the second physiological parameter meets a requirement for analyzing the second physiological parameter comprises:
determining whether the feature data of the second physiological parameter meets the requirement for analyzing the second physiological parameter; and
determining whether a data volume corresponding to the feature data of the second physiological parameter meets the requirement for analyzing the second physiological parameter when the feature data of the second physiological parameter meets the requirement for analyzing the second physiological parameter; and
the obtaining the second physiological parameter based on the feature data related to the second physiological parameter when the feature data of the second physiological parameter meets the requirement for analyzing the second physiological parameter comprises:
obtaining the second physiological parameter based on the feature data related to the second physiological parameter when the data volume corresponding to the feature data of the second physiological parameter meets the requirement for analyzing the second physiological parameter.

9. The method according to claim 8, wherein after the feature data of the second physiological parameter meets the requirement for analyzing the second physiological parameter, the method further comprises:
performing data processing on the feature data of the second physiological parameter; and
the determining whether a data volume corresponding to the feature data of the second physiological parameter meets the requirement for analyzing the second physiological parameter when the feature data of the second physiological parameter meets the requirement for analyzing the second physiological parameter comprises:
determining whether the data volume corresponding to the feature data of the second physiological parameter meets the requirement for analyzing the second physiological parameter when processed feature data of the second physiological parameter meets the requirement for analyzing the second physiological parameter.

10. The method according to any one of claims 1 to 9, after the measuring a first physiological parameter and a second physiological parameter of a measured object by using the physiological parameter sensor based on the physiological parameter measurement operation, the method further comprises:
displaying the first physiological parameter and the second physiological parameter of the measured object.

11. The method according to claim 10, wherein the displaying the first physiological parameter and the second physiological parameter of the measured object comprises:
displaying the first physiological parameter after measurement of the first physiological parameter is completed; and
displaying the second physiological parameter after measurement of the second physiological parameter is completed.

12. The method according to claim 10, wherein the displaying the first physiological parameter and the second physiological parameter of the measured object comprises:
displaying the first physiological parameter and the second physiological parameter after measurement of the first physiological parameter and the second physiological parameter is completed.

13. The method according to any one of claims 1 to 12, wherein the physiological parameter sensor comprises at least one of the following:
an accelerometer ACC sensor;
a photoplehysmography PPG sensor; and
an electrocardiography ECG sensor.

14. The method according to any one of claims 1 to 13, wherein the physiological parameter comprises at least one of the following:
atrial fibrillation;
premature beat;
a heart rate;
arteriosclerosis;
blood oxygen;
sleep; and
stress.

15. The method according to any one of claims 2 to 14, wherein the extracted data related to the first physiological parameter is partially or completely the same as the extracted data related to the second physiological parameter.

16. A system, wherein the system comprises a first application and a second application, wherein
the first application is used to collect data that is related to a first physiological parameter and that is of a measured object;
the first application is further configured to obtain the first physiological parameter based on the data related to the first physiological parameter;
the first application is further configured to detect an operation of measuring a second physiological parameter, wherein the second physiological parameter is different from the first physiological parameter;
the first application is further configured to send a measurement message to the second application in response to the operation, wherein the measurement message comprises the data related to the first physiological parameter; and
the second application is configured to obtain the second physiological parameter based on the data related to the first physiological parameter.

17. The system according to claim 16, wherein after the first application is further configured to obtain the first physiological parameter based on the data related to the first physiological parameter, the first application is further configured to:
determine the second physiological parameter based on the data related to the first physiological parameter.

18. A method for measuring a physiological parameter, wherein the method is applied to an electronic device comprising a physiological parameter sensor, and the method comprises:
collecting, by using the physiological parameter sensor, data that is related to a first physiological parameter and that is of a measured object;
obtaining the first physiological parameter based on the data related to the first physiological parameter;
detecting an operation of measuring a second physiological parameter, wherein the second physiological parameter is different from the first physiological parameter; and
sending a measurement message to a second application in response to the operation, wherein the measurement message comprises the data related to the first physiological parameter.

19. The method according to claim 18, wherein after the obtaining the first physiological parameter based on the data related to the first physiological parameter, the method further comprises:
determining the second physiological parameter based on the data related to the first physiological parameter.

20. A method for measuring a physiological parameter, wherein the method comprises:
receiving a measurement message from a first application, wherein the measurement message comprises data related to a first physiological parameter; and
obtaining a second physiological parameter based on the data related to the first physiological parameter, wherein the second physiological parameter is different from the first physiological parameter.

21. An electronic device, comprising one or more processors and one or more memories, wherein the one or more memories store one or more computer programs, the one or more computer programs comprise instructions, and when the instructions are executed by the one or more processors, the electronic device is enabled to perform the method for measuring a physiological parameter according to any one of claims 1 to 15, the electronic device is enabled to perform the method for measuring a physiological parameter according to claim 18 or 19, or the electronic device is enabled to perform the method for measuring a physiological parameter according to claim 20.

22. A computer readable storage medium, comprising computer instructions, wherein when the computer instructions are run on an electronic device, the electronic device is enabled to perform the method for measuring a physiological parameter according to any one of claims 1 to 15, the electronic device is enabled to perform the method for measuring a physiological parameter according to claim 18 or 19, or the electronic device is enabled to perform the method for measuring a physiological parameter according to claim 20.
